# EUROPEAN PATENT APPLICATION

(11) **EP 4 566 671 A2**
(43) Date of publication of application: **11.06.2025**
(21) Application number: 25168339.7
(22) Date of filing: 01.07.2021
(51) Int. Cl.: A61P 31/12

(54) **CONSTRUCTION AND APPLICATION OF FUSION PROTEIN VACCINE PLATFORM**

(30) Priority: 01.07.2020 CN 202010623708; 31.03.2021 CN 202110353488
(62) Divisional of application: 21833302.9
(71) Applicant: Institute of Biophysics Chinese Academy of Sciences, Chaoyang District Beijing 100101 (CN)
(72) Inventor: FU, Yangxin, Beijing, 100101 (CN); PENG, Hua, Beijing, 100101 (CN); SUN, Shiyu, Beijing, 100101 (CN)
(74) Representative: Nederlandsch Octrooibureau

(57) **Abstract**

The present invention relates to the construction and application of a fusion protein vaccine platform. The present invention provides a vaccine, comprising a fusion protein containing an interferon-target antigen-immunoglobulin Fc region (or antibody) and a Th cell helper epitope. The present invention also relates to use of a fusion protein containing an interferon-target antigen-immunoglobulin Fc region (or antibody) and a Th cell helper epitope in the preparation of prophylactic or therapeutic compositions. The vaccine of the present invention can be produced by eukaryotic cell expression systems to prepare wild-type and various mutant antigen vaccines, and vaccination by means of subcutaneous/muscular or nasal or other routes can lead to a strong immune response to a body. The vaccine of the present invention can be used as a prophylactic or therapeutic vaccine.

## Description

### FIELD OF THE INVENTION

The invention belongs to the field of genetic engineering and biomedical technology, and specifically relates to vaccines, for example, a vaccine comprising a fusion protein containing an interferon-target antigen-immunoglobulin Fc region (antibody) as framework. The vaccine of the present invention can be used as a vaccine platform for preventing hepatitis B virus (HBV) infection, human papilloma virus (HPV), Epstein-Barr virus (EBV), human immunodeficiency virus (HIV), severe acute respiratory syndrome coronavirus 2 (SARA-COV2), influenza virus infection and the develop of HPV, EBV-related tumors and for treating chronic hepatitis B (CHB) infection and HBV, HPV, and EBV-related tumors.

### BACKGROUND OF THE INVENTION

There are about 257 million chronic HBV infections in the world, and about 887,000 people die each year from end-stage liver diseases caused by HBV, including liver failure, liver cirrhosis, and hepatocellular carcinoma ^{[1-3]}. About 30% of liver cirrhosis is caused by HBV, and about 40% of hepatocellular carcinoma (HCC) is caused by HBV ^{[4]}. HBV infection remains a major public health problem worldwide. However, there is still no effective treatment strategy for chronic hepatitis B. The existing HBV treatment methods mainly include antiviral drugs (nucleoside/nucleotide analogs) and interferon. Although they have certain therapeutic effects, they usually cannot induce an effective immune response, so that HBV infection cannot be completely eliminated; moreover, long-term dosing may lead to significant side effects, and antiviral drugs will also lead to drug resistance. Chronic HBV infection is one of the main diseases that threaten human health. It is imminent to explore effective immunotherapy strategies for chronic hepatitis B. The development of therapeutic vaccines for chronic hepatitis B has very important social and economic significance.

Seasonal influenza causes severe illness in 1-4 million persons and kills 200,000-500,000 persons annually ^{[5]}. The best way to prevent and control influenza is through the vaccination, which reduces the incidence of illness and reduces the severity of infection, especially in young children and the elderly, who are at high risk of complications from influenza. Even though currently approved flu vaccines confer good protection effect in healthy young adults, there are still some issues that need to be addressed. For example, the production of some vaccines depends on chicken embryos, such as inactivated influenza vaccines and attenuated influenza vaccines. A disadvantage of these vaccines is that if the prevailing virus strains are of poultry origin, the epidemic of the disease will lead to an increase in demand for vaccines and chicken embryos, and thus leading to lack of chicken embryo supply ^{[6]}. Another disadvantage is that the production of these vaccines requires enormous amount of time. Elderly people are more prone to severe syndromes of influenza virus, and standard vaccines are generally not effective for the elderly, whose immune system gradually weakens with age ^{[7]}. In view of the problems encountered by current influenza vaccines, for the prevalence of influenza viruses, there is an urgent need for an influenza vaccine that has a strong immunogenicity, does not depend on chicken embryos, and can be produced quickly.

Severe acute respiratory syndrome coronavirus 2 (SARS-CoV-2) is a pathogen that caused the pandemic of 2019 coronavirus disease (COVID-19). The clinical symptoms caused by SARS-CoV-2 mainly include asymptomatic infection, mild flu-like symptoms, pneumonia and severe acute respiratory distress syndrome, which in severe cases may cause death in infected patients. At present, there is no specific medicine against SARS-CoV-2, and the vaccine is the basic countermeasure to control and end the SARS-CoV-2 pandemic ^{[9]}. In addition, the emergence of SARS-CoV-2 mutants poses new challenges to the existing candidate vaccines and the control of epidemic ^{[10]}. Therefore, powerful vaccines that are also effective for SARS-CoV-2 mutants are urgently needed in the current epidemic situation.

The linkage of an antigen to Fc region of an immunoglobulin will significantly increase the half-life of the antigen, and the Fc region of the immunoglobulin can bind to Fc receptors on the surface of antigen-presenting cells to promote the processing and presenting of the antigen by antigen-presenting cells ^{[11-13]}. Type I interferon has many biological activities as an antiviral cytokine, which includes the stimulation of immune cells ^{[14]}. IFNα can strongly induce the differentiation and activation of human DC cells ^{[15]}. Upon acting on immature DCs, type I interferon can promote the expression of MHC molecules and co-stimulatory molecules on the surface of DCs, such as MHC class I, CD80 and CD86, thereby enhancing the ability of DCs to activate T cells ^{[16-18]}. It has been reported that type I interferon can promote the antigen-presenting ability of DCs after infection with vaccinia virus and Lymphocytic ChorioMeningitis Virus (LCMV) ^{[19-21]}. In addition, type I interferon can promote the migration of DCs to lymph nodes by up-regulating the expression of chemokine receptors after acting on DCs, thereby promoting the activation of T cells ^{[22, 23]}. Recently, more and more studies have shown that type I interferon can be used as an immune adjuvant. The study by Le Bon et al. showed that when mice were immunized with a weak immunogen, type I interferon exhibited a strong immune adjuvant effect in mice and induced long-lasting antibodies and immune memory ^{[24]}, the author also found that the main cell populations in which type I interferon exerted its effect were DC cells. At the same time, antibodies are used to targeted deliver vaccines to DCs to stimulate DC activation and cross-presentation functions, which will further enhance the activity and potency of the vaccines.

There is a need for the present invention to provide a vaccine platform that enhances the response to viruses, bacteria or tumor antigens.

### SUMMARY OF THE INVENTION

Vaccines are an effective way to prevent and control major outbreaks of infectious diseases. There are various types of vaccines, one of which is protein subunit vaccines. In general, simple protein subunit vaccines generally have poor immunogenicity, which often limits the use of protein subunit vaccines. Therefore, a universal protein subunit vaccine platform is urgently needed. According to the impact of immunoglobulin Fc region and type I interferon on the immune system, the inventors propose a interferon alpha-viral antigen, bacteria or tumor-immunoglobulin Fc region fusion protein vaccine platform to enhance the immune response to viruses, bacteria or tumor antigens. The present invention provides a type I interferon-protein antigen-immunoglobulin Fc vaccine platform, wherein the type I interferon can promote antigen-presenting cells to allow maturation and migration so as to better play the role in antigen presentation and T cell activation. On the other hand, the Fc moiety of the vaccine platform can bind to the Fc receptors on the surface of antigen-presenting cells to enhance the uptake of antigens by antigen-presenting cells, thereby further enhancing antigen-presenting cells to function. The present inventors propose that the fusion of Th cell helper epitopes can further enhance the immune response effect of the vaccine of type I interferon-protein antigen-immunoglobulin Fc, and thus the Th cell helper epitope is an important element of the vaccine. The present inventors propose that anti-PD-L1 and other antibodies can be used to replace Fc, and the vaccine can be delivered to DCs to stimulate DC activation and cross-presentation, which will further enhance the activity and potency of the vaccine. As a novel vaccine platform, the vaccine platform of the present invention can be used as a prophylactic and therapeutic vaccine for diseases such as viral infections, bacterial infections or tumors.

In some embodiments, the present invention provides a vaccine comprising a fusion protein containing an interferon-target antigen-immunoglobulin Fc region (or antibody) (and an additional Th epitope). In some embodiments, the present invention also provides use of the fusion protein containing an interferon-target antigen-immunoglobulin Fc region (or antibody) (and an additional Th epitope) for the preparation of prophylactic or therapeutic compositions or kits (such as medicaments or vaccine compositions or kits). The vaccine of the present invention can be produced by eukaryotic cell expression systems, and inoculated by means of subcutaneous/muscular or intranasal or other immunization routes. For the fusion polypeptide of the present invention, the antibody (Ab for short) as a structural unit is not particularly limited, and may include, for example, a complete antibody or a fragment of antibody, such as an antibody heavy chain and light chain, or a single-chain antibody, and may be antibodies for DC targeting activation, including anti-PD-L1, anti-DEC205, anti-CD80/86 and other antibodies.

In some embodiments, the target antigen described herein is not particularly limited and may be any appropriate antigen. In some embodiments, the target antigens described herein can be, for example, tumor antigens and/or pathogen antigens (e.g., viral or bacterial antigens). In some embodiments, the target antigen described herein may be, for example, a tumor antigen, such as a protein molecule highly expressed by tumor cells, for example, human epidermal growth factor receptor 2 (HER2/neu), epidermal growth factor (EGFR).

In some embodiments, the target antigen used in the vaccine of the present invention can be, for example, a mutated target antigen that is different from the wild type. In some embodiments, the target antigen described herein can be, for example, mutants of tumor antigens and/or pathogen antigens such as viral or bacterial antigens. In some embodiments, the target antigen can be, for example, full length or S1 region of the S protein of SARS-COV-2 virus, for example, the target antigen can be the antigen as shown in SEQ ID NO.76 or SEQ ID NO.77. Herein, the wild-type target antigen refers to viruses or other infectious agents encoded by wild-type genes or immunogenic proteins expressed by tumors (the wild-type gene refers to the prevalent allele in nature, and is often used as a standard control gene in biological experiments), for example, Spike protein (S protein) derived from original wild-type strain of SARS-CoV-2. Herein, the mutated target antigen (mutant) refers to mutated viral proteins expressed by mutant virus strains and encoded by mutated gene derived from the wild-type genes, for example, the point mutations of S protein in different mutant SARS-CoV-2 that have been found include: 69-70 deletion, Y144 deletion, 242-244 deletion, L18F, D80A, D215, R246I mutation in NTD region, and K417, E484, N501Y mutation, L452R mutation, T478K mutation, D614G, H655Y mutation in RBD region. For example, these point mutations exist in different combinations in British SARS-CoV-2 B.1.1.7 (Alpha) mutant strains, South Africa B.1.351 (Beta) mutant strains, Brazil P1 (Gamma) mutant strains, India B.1.617, B.1.617.1 (Kappa), B.1.617.2 (Delta), B.1.617.3 mutant strains, California B.1.429 mutant strains and other mutant strains. In some embodiments, mutated target antigens may include for example natural point mutation/deletion mutation/addition mutation/truncation, artificial point mutation/deletion mutation/addition mutation/truncation, any combination of natural or artificial mutations, subtypes generated by mutations, wherein the target antigen may be a tumor antigen, a pathogen antigen, such as a virus (e.g., SARS-COV-2) or a bacterial antigen. In some embodiments, the target antigen used in the vaccine of the present invention is a mutated viral antigen, for example, the mutated viral antigen can be a mutant of SARS-COV-2, including for example natural point mutation/deletion mutation/addition mutation/truncation, artificial point mutation/deletion mutation/addition mutation/truncation, any combination of natural or artificial mutations, subtypes generated by mutations, derived from SARS-COV-2 protein (such as one or more of S protein, N protein, M protein, E protein); for example, the mutated viral antigen can be a mutant of the full length of S protein, the S1 region, and the RBD region; for example, the mutated viral antigen may include one or more of the following mutations in S protein of SARS-COV-2: 69-70 deletion, Y144 deletion, 242-244 deletion, L18F, D80A, D215, R246I mutation in NTD region, K417,, E484, N501Y mutation, L452R mutation, T478K mutation , D614G, H655Y mutation in RBD region; for example, the mutated viral antigen may include mutations present in the British B.1.1.7 (Alpha) mutant strain, the South Africa B.1.351 (Beta) mutant strain and the Brazil P1 (Gamma) mutant strain, India B.1.617, B.1.617.1 (Kappa), B.1.617.2 (Delta), B.1.617.3 mutants, and California B.1.429 mutant; for example, the mutated viral antigen may contain a mutant shown in any one of SEQ ID NO.79, SEQ ID NO.80, and SEQ ID NO.81; the mutated viral antigen may contain a mutant comprising a sequence shown in any one of SEQ ID NO.79, SEQ ID NO.80, SEQ ID NO.81, SEQ ID NO.82, SEQ ID NO.83, and SEQ ID NO.84. Herein, unless otherwise clearly stated or clearly limited by the context, the target antigen herein generally includes wild-type target antigens and mutant target antigens.

The object of the present invention is to provide a vaccine platform, which consists of an interferon (IFN) and a tumor, bacterium or virus antigen (hepatitis B virus Pres1 antigen, SARS-COV2 RBD antigen, influenza HA antigen, human papillomavirus HPV E7 antigen, hepatitis B virus surface antigen (HBsAg) antigen or peptide fragment, herpes zoster virus (VZV) gE antigen, Epstein-Barr virus (EBV) EBNA1/LMP2/gp350, herpes simplex virus 2 (HSV-2) gD antigen, HIV gp120 antigen-immunoglobulin Fc region (or antibody) (and an additional Th epitope). The fusion protein can be a homodimeric or heterodimeric protein. In the case that the fusion protein is in the form of a dimer, the interferon, the target antigen, and the immunoglobulin Fc region (or antibody Ab) as structural units can exist in the first polypeptide chain and/or the second polypeptide chain, and the existence of each structural unit is not particularly limited, for example, they can all exist in one chain, or any one or more structural units can exist in one chain, while other one or more structural units can exist in another chain.

The interferon of the present invention can be selected from type I interferon, type II interferon and type III interferon, such as IFN-α, IFN-β, IFN-γ, IFN-λ1 (IL-29), IFN-λ2 (IL - 28a), IFN-λ (IL-28b) and IFN-ω; the IFN can be derived from human or mouse; preferably type I interferon IFN-α (SEQ ID NO.1, SEQ ID NO.21, SEQ ID NO.22).

The immunoglobulin Fc region of the present invention can be selected from the constant region amino acid sequences of IgG1, IgG2, IgG3 and IgG4/or IgM, preferably IgG1 (SEQ ID NO.2, SEQ ID NO.23, SEQ ID NO.24).

The fusion polypeptide of the present invention may also optionally comprise one or more Th cell helper epitopes and/or linking fragments (linkers). For example, when the fusion protein is in the form of a dimer, optionally the fusion protein can also comprises one or more Th cell helper epitopes and/or linking fragments in any one or two chains of the homodimer or heterodimer (i.e. the first polypeptide chain and/or or the second polypeptide chain). As known to those skilled in the art, the various structural units of the fusion protein can be connected by appropriate linking fragments (linkers). The linking fragments that can be used in the vaccine of the present invention are not particularly limited, and can be any suitable peptide fragments known in the art. The linking fragments of each structural unit in the present invention can be flexible polypeptide sequences, and can be linking fragments 1 and 2, for example as shown in the amino acid sequences of SEQ ID NO.4 and SEQ ID NO.25.

The N-terminal of the polypeptide sequence composed of each structural unit in the present invention contains a corresponding signal peptide capable of promoting protein secretion, for example as shown in the amino acid sequence of SEQ ID NO.5.

Preferred antigens described in the present invention include hepatitis B Pres1 antigen, including ad subtype (SEQ ID NO.6) ,ay subtype (SEQ ID NO.26), HBV HBsAg antigen (various subtypes and peptide fragments), including adr subtype (SEQ ID NO.7), adw subtype (SEQ ID NO.27), ayw subtype (SEQ ID NO.28), SARS-COV2 RBD antigen (SEQ ID NO.8), influenza virus HA antigen (SEQ ID NO.9), HPV E7 antigen (SEQ ID NO.10); herpes virus VZV-gE antigen (SEQ ID NO.91), EBV-gp350 antigen (SEQ ID NO.92), HSV-2-gD antigen (SEQ ID NO.93).

The homodimeric protein described in the present invention comprises a first polypeptide and a second polypeptide, and the first polypeptide and the second polypeptide are completely identical. The order of the elements from N-terminal to C-terminal in the first polypeptide and the second polypeptide is IFN-tumor or virus antigen (hepatitis B Pres1 antigen, SARS-COV2 RBD antigen, influenza HA antigen, HPV E7 antigen, HBsAg antigen, VZV-gE antigen, EBV EBNA1/LMP2/gp350, HSV-2-gD antigen, HIV gp120 antigen) - immunoglobulin Fc region; or a polypeptide containing a Pan epitope. The homodimeric protein of the present invention comprises the a sequences as shown in SEQ ID NO.11, 12, 13, 14, 29, 30, 31, 32, 38, 39, 40, 47, 48, 49, 50, 51, 56, 57, 59, 58, 65, 66, 67, or 68.

The heterodimer of the present invention comprises a first polypeptide and a second polypeptide, wherein the first polypeptide and the second polypeptide are not identical; the first polypeptide, from the C terminal to the N terminal, is respectively IFN-immunoglobulin Fc region, and comprises an amino acid sequence as shown in SEQ ID NO.15, 33, 42, 51, 60, or 69; the second polypeptide, from the C terminal to the N terminal, is respectively a tumor or virus antigen (Hepatitis B Pres1 antigen, SARS-COV2 RBD antigen, influenza HA antigen, HPV E7 antigen, VZV-gE antigen, EBV EBNA1/LMP2/gp350, HSV-2-gD antigen, HIV gp120 antigen) - immunoglobulin Fc region, and comprises an amino acid sequence as shown in SEQ ID NO. 16, 17, 18, 19, 34, 35, 36, 37, 43, 44, 45, 46, 52, 53, 54, 55, 61, 62, 63, 64, 70, 71, 72, or 73.

The present invention also provides a nucleotide sequence encoding the above IFN-tumor or virus antigen (hepatitis B Pres1 antigen, HBsAg antigen or peptide, SARS-COV2 RBD antigen, influenza HA antigen, HPV E7 antigen, VZV-gE antigen, EBV EBNA1/LMP2/gp350, HSV- 2-gD antigen, HIV gp120 antigen)-immunoglobulin Fc vaccine platform.

The present invention also relates to a nucleotide fragment encoding the vaccine platform and fusion protein.

The present invention also relates to a preparation method of the fusion protein or vaccine platform, for example, the preparation method includes the following steps:
(1) An expression vector comprising the gene encoding the fusion protein or vaccine platform is constructed; preferably, the expression vector is a pEE12.4 expression vector;
(2) A host cell comprising the expression vector is constructed by transient transfection; preferably, the host cell is a 293F cell;
(3) The host cells are cultured and cell supernatant is collected;
(4) The fusion protein or vaccine platform is purified by protein A/G affinity chromatography column.

The present invention also includes the application of the vaccine platform; the vaccine platform can be used as a prophylactic vaccine for hepatitis B, a therapeutic vaccine for hepatitis B, a prophylactic vaccine for influenza, a prophylactic vaccine for SARA-COV2, influenza, HPV, VZV, EBV, HSV-2, and HIV, and a prophylactic vaccine for HPV and EBV-related tumors.

The present invention includes adjuvants used in the vaccine platform, wherein the adjuvants include aluminum adjuvant (Alum), Toll-like receptor 4 activator ligand MPLA, Toll-like receptor 9 ligand, M59, oligodeoxy Nucleotides (CpG-ODN) and Freund's adjuvant.

The present invention includes the clinical use of the vaccine platform as an HBV therapeutic vaccine in combination with hepatitis B virus envelope protein HBsAg vaccine in the treatment of chronic hepatitis B virus infection.

The present invention includes the clinical use of the vaccine platform as an HBV therapeutic vaccine in combination with nucleoside or nucleotide analogues in the treatment of chronic hepatitis B virus infection.

The present invention includes combined application of the vaccine platform as a prophylactic or therapeutic vaccine for HBV, influenza, SARS-COV2, HPV, VZV, EBV, HSV-2, and HIV in combination with antiviral drugs and other therapies; as a prophylactic or therapeutic vaccine for HBV, HPV, and EBV-related tumors in combination with antiviral and antitumor drugs and therapies.

The present invention comprises multivalent combination vaccine consisting of the vaccine platform and other virus or pathogen or tumor vaccines.

Any fusion protein vaccine comprising the vaccine platform of the present invention can be inoculated with the adenovirus vaccine, mRNA vaccine, inactivated vaccine or DNA vaccine for the same virus, pathogen or tumor in sequence or simultaneously.

The present invention includes the full-length sequence and any truncation sequence of the vaccine platform antigen, such as SEQ ID NO.76, SEQ ID NO.77, SEQ ID NO.78.

The present invention comprises any possible mutants of said fusion protein vaccine antigen, including natural point mutation/deletion mutation/truncation, any combination of natural sit mutations, subtypes generated by mutations, and mutated sequences comprising artificial point mutation/deletion mutation/truncation constructed for the purpose of enhancing the effect of the vaccine, such as SEQ ID NO.79, SEQ ID NO.80, SEQ ID NO.81, SEQ ID NO.82, SEQ ID NO. 83 , SEQ ID NO.84.

The present invention provides a multivalent combination vaccine consisting any vaccine of the present invention as a component of the vaccine and another vaccine of the present invention or other vaccines different from the vaccine of the present invention such as other virus or pathogen or tumor vaccines, for example, a multivalent vaccine comprising the SARS-CoV-2 fusion protein vaccine of the present invention in combination with influenza vaccine or other vaccines; for example, any vaccine of the present invention and the adenovirus vaccine or mRNA vaccine or inactivated vaccine or DNA vaccine for the same virus, pathogen, or tumor can be inoculated in sequence or simultaneously; for example, the SARS-COV-2 fusion protein vaccine can be inoculated with the adenovirus vaccine, or mRNA vaccine, or inactivated vaccine or DNA vaccine for SARS-COV-2 in sequence or simultaneously; for example, the sequence for immunization may be as follows: 1) firstly immunization with the SARS-COV-2 fusion protein vaccine of the present invention, and then immunization with the adenovirus vaccine or mRNA vaccine or inactivated vaccine or DNA vaccine of SARS-COV-2; 2) firstly immunization with the adenovirus vaccine or mRNA vaccine or inactivated vaccine or DNA vaccine of SARS-COV-2, followed by immunization with the SARS-COV-2 fusion protein vaccine; 3) the SARS-COV-2 fusion protein vaccine and the adenovirus vaccine or mRNA vaccine or inactivated vaccine or DNA vaccine are inoculated simultaneously. As known in the art, in the case of combination use, the vaccines can be prepared as a convenient kit.

The present invention includes but not limited to the following advantages over the prior art:
1. The antigens of the IFN-tumor or virus antigen-immunoglobulin Fc (or antibody) vaccine platform provided by the present invention can be varied in various components, either tumor-associated antigens or virus-specific antigens, which enhances the flexibility of the vaccine platform, as well as the scope of use of the vaccine platform.
2. In the IFN-tumor or virus antigen-immunoglobulin Fc (or antibody) vaccine platform provided by the present invention, the interferon (IFN) can enhance the migration and maturation of antigen-presenting cells, and increase the expression of costimulatory factors, thereby promoting the presentation of antigens to T cells; meanwhile, the Fc region (or antibody) in the vaccine platform, on the one hand, increases the molecular weight of the antigen and thus increases the half-life thereof, and on the other hand, the Fc region (or antibody) can bind to Fc receptors on the surface of antigen-presenting cells and promote the processing and presentation of antigens by antigen-presenting cells, thereby promoting the generation of immune responses.
3. The IFN-tumor or virus antigen-immunoglobulin Fc (or antibody) vaccine platform provided by the present invention is expressed by eukaryotic HEK293 cell expression system, and the proteins expressed by HEK293 cells are closer to natural protein molecules either in molecular structure or in physical and chemical characteristics and protein modification and biological functions of proteins.
4. The IFN-tumor or virus antigen-immunoglobulin Fc (or antibody) vaccine platform provided by the present invention may be in the form of homodimer or heterodimer, and has better choices for different antigens.
5. The IFN-tumor or virus antigen-immunoglobulin Fc vaccine platform provided by the present invention can activate DC to enhance DC cross-presentation and generate strong B cell and T cell immune responses by fusing Th cell helper epitopes such as Pan epitopes, using DC targeting antibodies such as anti-PD-L1, and adding various adjuvants to stimulate immune responses.
6. The IFN-tumor or virus antigen-immunoglobulin Fc (or antibody) vaccine platform provided by the present invention has a wide range of applications and can be used not only as a prophylactic vaccine, but also as a therapeutic vaccine.
7. The IFN-tumor or virus antigen-immunoglobulin Fc (or antibody) vaccine platform provided by the present invention can not only be used alone, but also can be used as a therapeutic vaccine in combination with existing commercial HBsAg vaccines and nucleoside/nucleotide analogues.
8. The vaccine platform provided by the present invention can be used in combination with other virus or pathogen or tumor vaccines to form a multivalent vaccine.
9. Any fusion protein vaccine in the vaccine platform of the present invention can be inoculated together with the adenovirus vaccine, mRNA vaccine, inactivated vaccine or DNA vaccine for the same virus, pathogen or tumor in sequence or simultaneously.
10. The present invention includes the full-length sequence and any truncation sequence of the vaccine platform antigen.
11. Any possible mutants of the vaccine platform antigen provided in the present invention includes natural point mutation/deletion mutation/addition mutation/truncation, any combination of natural point mutations, subtypes generated by mutations, and mutated sequences comprising artificial point mutation/deletion mutation/addition mutation/truncation constructed for the purpose of enhancing the effect of the vaccine.

### Sequences involved in the present invention:

1. Unit component sequence:
   SEQ ID NO.1: Amino acid sequence of mouse mIFNα4 (mIFNα) 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKE
 SEQ ID NO.21: Amino acid sequence of human IFNα2 (hIFNα) SEQ ID.NO.22: Amino acid sequence of human mutant IFNα2 (Q124R) (hmIFNα) 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKE
 SEQ ID NO.2: Amino acid sequence of human IgG1-Fc 
EPKSCDKTHTCPPCPAPELLGGPSVFLFPPKPKDQLMISRTPEVTCVVVDVSHED
PEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQ
PENNYKTTPPVLDSDGSFLYSKLTVDKSRWQQGNVFSCSVLHEALHNHYTQKSLSLSP
GKHV
   SEQ ID No.23: Heterodimer Fc-hole 
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVCTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPGK
   SEQ ID No.24: Heterodimer Fc-knob 
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPCRDELTKNQVSLWCLVKGFYPSDIAVEWESNGQPENN
YKTTPPVLDSDGSFFLYSKLTVDKSRWQQGNVFSCSVMHEALHNHYTQKSLSLSPGK
   SEQ ID NO.3: Amino acid sequence of Th helper epitope Pan HLA DR-binding epitope (PADER)
      AKFVAAWTLKAAA
   SEQ ID NO.4: Amino acid sequence of Linker 1:
      GGGGSGGGGSGGGGS
   SEQ ID NO.25: Amino acid sequence of Linker 2:
      GSGSGS
   SEQ ID NO.5: Amino acid sequence of Signal peptide:
      MARLCAFLMILVMMSYYWSACSLG
   SEQ ID NO.6: Amino acid sequence of HBV Pres1 (ad subtype) 
MGGWSSKPRKGMGTNLSVPNPLGFFPDHQLDPAFGANSNNPDWDFNPIKDHW
PAANQVGVGAFGPGLTPPHGGILGWSPQAQGILTTVSTIPPPASTNRQSGRQPTPISPPLR
DSHPQA
   SEQ ID NO.26: Amino acid sequence of HBV Pres1 (ay subtype) 
MGQNLSTSNPLGFFPDHQLDPAFRANTANPDWDFNPNKDTWPDANKVGAGAF
GLGFTPPHGGLLGWSPQAQGILQTLPANPPPASTNRQTGRQPTPLSPPLRNTHPQA
   SEQ ID NO.7: Amino acid sequence of HBV HBsAg (adr subtype) 
MENTTSGFLGPLLVLQAGFFLLTRILTIPQSLDSWWTSLNFLGGAPTCPGQNSQS
PTSNHSPTSCPPICPGYRWMCLRRFIIFLFILLLCLIFLLVLLDYQGMLPVCPLLPGTSTTS
TGPCKTCTIPAQGTSMFPSCCCTKPSDGNCTCIPIPSSWAFARFLWEWASVRFSWLSLLV
PFVQWFVGLSPTVWLSVIWMMWYWGPSLYNILSPFLPLLPIFFCLWVYI
   SEQ ID NO.27: Amino acid sequence of HBV HBsAg (adw subtype) 
MENITSGLLGPLLVLQAGFFLLTRILTIPQSLDSWWTSLSFLGEAPVCLGQNSQSP
TRNHSPTSCPPICPGYRWMCLRRFIIFLFILLLCLIFLLVLLDYQGMLPVCPLIPGSTTTST
GPCKTCTTPAQGNSMFPSCCCTKPTDGNCTCIPIPSSWAFAKYLWEWASVRFSWLSLL
VPFVQWFVGLSPTVWLSAIWMIWYWGPSLYSIVCPFTPLLQIFCCLWVFI
   SEQ ID NO.28: Amino acid sequence of HBV HBsAg (ayw subtype) 
MENITSGFLGPLLVLQAGFFLLTRILTIPQSLDSWWTSLNFLGGTTVCLGQSSQSP
TSNHSPTSCPPTCPGYRWMCLRRFIIFLFILLLCLIFLLVLLDYQGMLPVCPLIPGSSTTST
GPCRTCMTTAQGTSMYPSCCCTKPSDGNCTCIPIPSSWAFGKFLWEWASARFSWLSLL
VPFVQWFVGLSPTVWLSVIWMMWYWGPSLYSILSPFLPLLPIFFCLWVYI
   SEQ ID NO.8: Amino acid sequence of SARS-CoV-2 RBD 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNF
   SEQ ID NO.9: Amino acid sequence of influenza HA: 
DTICIGYHANNSTDTVDTVLEKNVTVTHSVNLLEDSHNGKLCRLKGIAPLQLGK
CNIAGWLLGNPECDPLLPVRSWSYIVETPNSENGICYPGDFIDYEELREQLSSVSSFERFE
IFPKESSWPNHNTNGVTAACSHEGKSSFYRNLLWLTEKEGSYPKLKNSYVNKKGKEVL
 
VLWGIHHPPNSKEQQNLYQNENAYVSVVTSNYNRRFTPEIAERPKVRDQAGRMNYYW
TLLKPGDTIIFEANGNLIAPMYAFALSRGFGSGIITSNASMHECNTKCQTPLGAINSSLPY
QNIHPVTIGECPKYVRSAKLRMVTGLRNNPSIQSR
   SEQ ID NO.10: Amino acid sequence of HPV-E7 antigen 
MHGDTPTLHEYMLDLQPETTDLYCYEQLNDSSEEEDEIDGPAGQAEPDRAHYNI
VTFCCKCDSTLRLCVQSTHVDIRTLEDLLMGTLGIVCPICSQKP
2. Sequences of Murine IFN vaccine mIFNα-antigen-Fc:
   SEQ ID NO.11: Amino acid sequence of mIFNα-Pres1-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTMGQNLSTSNPLGFFPDHQLDPAFRANTANPDWDFNPNKDTWP
DANKVGAGAFGLGFTPPHGGLLGWSPQAQGILQTLPANPPPASTNRQTGRQPTPLSPPL
RNTHPQAFEEPKSCDKTHTCPPCPAPELLGGPSVFLFPPKPKDQLMISRTPEVTCVVVDV
SHEDPEVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIAVEWE
SNGQPENNYKTTPPVLDSDGSFLYSKLTVDKSRWQQGNVFSCSVLHEALHNHYTQKSL
SLSPGKHV
   SEQ ID NO.12: Amino acid sequence of mIFNα-RBD (SARS-CoV-2)-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTRVQPTESIVRFPNITNLCPFGEVFNATRFASVYAWNRKRISNCVA
DYSVLYNSASFSTFKCYGVSPTKLNDLCFTNVYADSFVIRGDEVRQIAPGQTGKIADYN
YKLPDDFTGCVIAWNSNNLDSKVGGNYNYLYRLFRKSNLKPFERDISTEIYQAGSTPCN
GVEGFNCYFPLQSYGFQPTNGVGYQPYRVVVLSFELLHAPATVCGPKKSTNLVKNKC
VNFFEEPKSCDKTHTCPPCPAPELLGGPSVFLFPPKPKDQLMISRTPEVTCVVVDVSHED
PEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQ
PENNYKTTPPVLDSDGSFLYSKLTVDKSRWQQGNVFSCSVLHEALHNHYTQKSLSLSP
GKHV
   SEQ ID NO.13: Amino acid sequence of mIFNα-HA-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTDTICIGYHANNSTDTVDTVLEKNVTVTHSVNLLEDSHNGKLCRL
 
KGIAPLQLGKCNIAGWLLGNPECDPLLPVRSWSYIVETPNSENGICYPGDFIDYEELREQ
LSSVSSFERFEIFPKESSWPNHNTNGVTAACSHEGKSSFYRNLLWLTEKEGSYPKLKNS
YVNKKGKEVLVLWGIHHPPNSKEQQNLYQNENAYVSVVTSNYNRRFTPEIAERPKVR
DQAGRMNYYWTLLKPGDTIIFEANGNLIAPMYAFALSRGFGSGIITSNASMHECNTKC
QTPLGAINSSLPYQNIHPVTIGECPKYVRSAKLRMVTGLRNNPSIQSRFEEPKSCDKTHT
CPPCPAPELLGGPSVFLFPPKPKDQLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFLYSKLTVDKSRWQQGNVFSCSVLHEALHNHYTQKSLSLSPGKHV
   SEQ ID NO.14: Amino acid sequence of mIFNα-E7 (HPV)-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTMHGDTPTLHEYMLDLQPETTDLYCYEQLNDSSEEEDEIDGPAG
QAEPDRAHYNIVTFCCKCDSTLRLCVQSTHVDIRTLEDLLMGTLGIVCPICSQKPFEEPK
SCDKTHTCPPCPAPELLGGPSVFLFPPKPKDQLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKT
TPPVLDSDGSFLYSKLTVDKSRWQQGNVFSCSVLHEALHNHYTQKSLSLSPGKHV
   SEQ ID NO.15: Amino acid sequence of the first chain mIFNα-Fc-hole in heterodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKC
KVSNKALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYT
QKSLSLSPGK
   SEQ ID NO.16: Amino acid sequence of the second chain Pres1-Fc-knob in heterodimer mIFNα-Pres1-Fc 
MGQNLSTSNPLGFFPDHQLDPAFRANTANPDWDFNPNKDTWPDANKVGAGAF
GLGFTPPHGGLLGWSPQAQGILQTLPANPPPASTNRQTGRQPTPLSPPLRNTHPQAFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK
   SEQ ID NO.17: Amino acid sequence of the second chain RBD (SARS-CoV-2)-Fc-knob in heterodimer mIFNα-RBD (SARA-CoV-2)-Fc 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNFFEDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPCRDELTKNQVSLWCLVKGFYPSDIAVEWESNGQPENNYKTTPPVLDSD
GSFFLYSKLTVDKSRWQQGNVFSCSVMHEALHNHYTQKSLSLSPGK
   SEQ ID NO.18: Amino acid sequence of the second chain HA-Fc-knob in heterodimer mIFNα-HA-Fc 
DTICIGYHANNSTDTVDTVLEKNVTVTHSVNLLEDSHNGKLCRLKGIAPLQLGK
CNIAGWLLGNPECDPLLPVRSWSYIVETPNSENGICYPGDFIDYEELREQLSSVSSFERFE
IFPKESSWPNHNTNGVTAACSHEGKSSFYRNLLWLTEKEGSYPKLKNSYVNKKGKEVL
VLWGIHHPPNSKEQQNLYQNENAYVSVVTSNYNRRFTPEIAERPKVRDQAGRMNYYW
TLLKPGDTIIFEANGNLIAPMYAFALSRGFGSGIITSNASMHECNTKCQTPLGAINSSLPY
QNIHPVTIGECPKYVRSAKLRMVTGLRNNPSIQSRFEDKTHTCPPCPAPELLGGPSVFLF
PPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPCRDEL
TKNQVSLWCLVKGFYPSDIAVEWESNGQPENNYKTTPPVLDSDGSFFLYSKLTVDKSR
WQQGNVFSCSVMHEALHNHYTQKSLSLSPGK
   SEQ ID NO.19: Amino acid sequence of the second chain E7-Fc-knob in heterodimer mIFNα-E7 (HPV)-Fc 
MHGDTPTLHEYMLDLQPETTDLYCYEQLNDSSEEEDEIDGPAGQAEPDRAHYNI
VTFCCKCDSTLRLCVQSTHVDIRTLEDLLMGTLGIVCPICSQKPFEDKTHTCPPCPAPEL
LGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGK
3. Sequences of Murine IFN and Pan epitope-containing vaccine IFNα-Pan-antigen-Fc:
   SEQ ID NO.29: Amino acid sequence of mIFNα-Pan -Pres1-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKEGGGGSGGG
GSGGGGSRTAKFVAAWTLKAAAGSGSGSMGQNLSTSNPLGFFPDHQLDPAFRANTAN
PDWDFNPNKDTWPDANKVGAGAFGLGFTPPHGGLLGWSPQAQGILQTLPANPPPAST
 
NRQTGRQPTPLSPPLRNTHPQAFEDKTHTCPPCPAPELLGGPSVFLFPPKPKDQLMISRT
PEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVK
GFYPSDIAVEWESNGQPENNYKTTPPVLDSDGSFLYSKLTVDKSRWQQGNVFSCSVLH
EALHNHYTQKSLSLSPGK
   SEQ ID NO.30: Amino acid sequence of mIFNα-Pan -RBD (SARS-CoV-2)-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKEGGGGSGGG
GSGGGGSRTAKFVAAWTLKAAAGSGSGSRVQPTESIVRFPNITNLCPFGEVFNATRFAS
VYAWNRKRISNCVADYSVLYNSASFSTFKCYGVSPTKLNDLCFTNVYADSFVIRGDEV
RQIAPGQTGKIADYNYKLPDDFTGCVIAWNSNNLDSKVGGNYNYLYRLFRKSNLKPFE
RDISTEIYQAGSTPCNGVEGFNCYFPLQSYGFQPTNGVGYQPYRVVVLSFELLHAPATV
CGPKKSTNLVKNKCVNFFEDKTHTCPPCPAPELLGGPSVFLFPPKPKDQLMISRTPEVTC
VVVDVSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGFYPSDI
AVEWESNGQPENNYKTTPPVLDSDGSFLYSKLTVDKSRWQQGNVFSCSVLHEALHNH
YTQKSLSLSPGK
   SEQ ID NO.31: Amino acid sequence of mIFNα-Pan -HA-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKEGGGGSGGG
GSGGGGSRTAKFVAAWTLKAAAGSGSGSDTICIGYHANNSTDTVDTVLEKNVTVTHS
VNLLEDSHNGKLCRLKGIAPLQLGKCNIAGWLLGNPECDPLLPVRSWSYIVETPNSENG
ICYPGDFIDYEELREQLSSVSSFERFEIFPKESSWPNHNTNGVTAACSHEGKSSFYRNLL
WLTEKEGSYPKLKNSYVNKKGKEVLVLWGIHHPPNSKEQQNLYQNENAYVSVVTSNY
NRRFTPEIAERPKVRDQAGRMNYYWTLLKPGDTIIFEANGNLIAPMYAFALSRGFGSGII
TSNASMHECNTKCQTPLGAINSSLPYQNIHPVTIGECPKYVRSAKLRMVTGLRNNPSIQ
SRFEDKTHTCPPCPAPELLGGPSVFLFPPKPKDQLMISRTPEVTCVVVDVSHEDPEVKFN
WYVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENNY
KTTPPVLDSDGSFLYSKLTVDKSRWQQGNVFSCSVLHEALHNHYTQKSLSLSPGK
   SEQ ID NO.32: Amino acid sequence of mIFNα-Pan -E7 (HPV)-Fc in homodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKEGGGGSGGG
 
GSGGGGSRTAKFVAAWTLKAAAGSGSGSMHGDTPTLHEYMLDLQPETTDLYCYEQL
NDSSEEEDEIDGPAGQAEPDRAHYNIVTFCCKCDSTLRLCVQSTHVDIRTLEDLLMGTL
GIVCPICSQKPFEDKTHTCPPCPAPELLGGPSVFLFPPKPKDQLMISRTPEVTCVVVDVSH
EDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESN
GQPENNYKTTPPVLDSDGSFLYSKLTVDKSRWQQGNVFSCSVLHEALHNHYTQKSLSL
SPGK
   SEQ ID NO.33: Amino acid sequence of the first chain mIFNα-Fc-hole in heterodimer 
CDLPHTYNLGNKRALTVLEEMRRLPPLSCLKDRKDFGFPLEKVDNQQIQKAQAI
LVLRDLTQQILNLFTSKDLSATWNATLLDSFCNDLHQQLNDLKACVMQEPPLTQEDSL
LAVRTYFHRITVYLRKKKHSLCAWEVIRAEVWRALSSSTNLLARLSEEKESGGGGSGG
GGSGGGGSGGGGRTDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKC
KVSNKALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYT
QKSLSLSPGK
   SEQ ID NO: 34: Amino acid sequence of the second chain Pan-Pres1-Fc-knob in heterodimer mIFN-Pan-Pres 1-Fc 
AKFVAAWTLKAAAGSGSGSMGQNLSTSNPLGFFPDHQLDPAFRANTANPDWD
FNPNKDTWPDANKVGAGAFGLGFTPPHGGLLGWSPQAQGILQTLPANPPPASTNRQTG
RQPTPLSPPLRNTHPQAFEDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKE
YKCKVSNKALPAPIEKTISKAKGQPREPQVYTLPPCRDELTKNQVSLWCLVKGFYPSDI
AVEWESNGQPENNYKTTPPVLDSDGSFFLYSKLTVDKSRWQQGNVFSCSVMHEALHN
HYTQKSLSLSPGK
   SEQ ID NO.35: Amino acid sequence of the second chain Pan-RBD (SARS-CoV-2)-Fc-knob in heterodimer mIFNα-Pan-RBD (SARA-CoV-2)-Fc 
AKFVAAWTLKAAAGSGSGSRVQPTESIVRFPNITNLCPFGEVFNATRFASVYAW
NRKRISNCVADYSVLYNSASFSTFKCYGVSPTKLNDLCFTNVYADSFVIRGDEVRQIAP
GQTGKIADYNYKLPDDFTGCVIAWNSNNLDSKVGGNYNYLYRLFRKSNLKPFERDIST
EIYQAGSTPCNGVEGFNCYFPLQSYGFQPTNGVGYQPYRVVVLSFELLHAPATVCGPK
KSTNLVKNKCVNFFEDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVYTLPPCRDELTKNQVSLWCLVKGFYPSDIAV
EWESNGQPENNYKTTPPVLDSDGSFFLYSKLTVDKSRWQQGNVFSCSVMHEALHNHY
TQKSLSLSPGK
   SEQ ID NO.36: Amino acid sequence of the second chain Pan-HA-Fc-knob in heterodimer mIFNα-Pan-HA-Fc 
AKFVAAWTLKAAAGSGSGSDTICIGYHANNSTDTVDTVLEKNVTVTHSVNLLE
DSHNGKLCRLKGIAPLQLGKCNIAGWLLGNPECDPLLPVRSWSYIVETPNSENGICYPG
DFIDYEELREQLSSVSSFERFEIFPKESSWPNHNTNGVTAACSHEGKSSFYRNLLWLTEK
EGSYPKLKNSYVNKKGKEVLVLWGIHHPPNSKEQQNLYQNENAYVSVVTSNYNRRFT
PEIAERPKVRDQAGRMNYYWTLLKPGDTIIFEANGNLIAPMYAFALSRGFGSGIITSNAS
MHECNTKCQTPLGAINSSLPYQNIHPVTIGECPKYVRSAKLRMVTGLRNNPSIQSRFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK
   SEQ ID NO.37: Amino acid sequence of the second chain Pan-E7-Fc-knob in heterodimer mIFNα-Pan-E7 (HPV)-Fc 
AKFVAAWTLKAAAGSGSGSMHGDTPTLHEYMLDLQPETTDLYCYEQLNDSSE
EEDEIDGPAGQAEPDRAHYNIVTFCCKCDSTLRLCVQSTHVDIRTLEDLLMGTLGIVCPI
CSQKPFEDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPCRDELTKNQVSLWCLVKGFYPSDIAVEWESNGQPE
NNYKTTPPVLDSDGSFFLYSKLTVDKSRWQQGNVFSCSVMHEALHNHYTQKSLSLSPG
K
4. Sequences of Human IFN vaccine hIFNα-antigen-Fc:
   SEQ ID NO.38: Amino acid sequence of hIFNα-Pres1-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEGGGGSGG
GGSGGGGSRTMGQNLSTSNPLGFFPDHQLDPAFRANTANPDWDFNPNKDTWPDANK
VGAGAFGLGFTPPHGGLLGWSPQAQGILQTLPANPPPASTNRQTGRQPTPLSPPLRNTH
PQAFEDKTHTCPPCPAPELLGGPSVFLFPPKPKDQLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENN
YKTTPPVLDSDGSFLYSKLTVDKSRWQQGNVFSCSVLHEALHNHYTQKSLSLSPGK
   SEQ ID NO.39: Amino acid sequence of hIFNα-RBD (SARS-CoV-2)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEGGGGSGG
GGSGGGGSRTRVQPTESIVRFPNITNLCPFGEVFNATRFASVYAWNRKRISNCVADYSV
 
LYNSASFSTFKCYGVSPTKLNDLCFTNVYADSFVIRGDEVRQIAPGQTGKIADYNYKLP
DDFTGCVIAWNSNNLDSKVGGNYNYLYRLFRKSNLKPFERDISTEIYQAGSTPCNGVE
GFNCYFPLQSYGFQPTNGVGYQPYRVVVLSFELLHAPATVCGPKKSTNLVKNKCVNFF
EDKTHTCPPCPAPELLGGPSVFLFPPKPKDQLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKTT
PPVLDSDGSFLYSKLTVDKSRWQQGNVFSCSVLHEALHNHYTQKSLSLSPGK
   SEQ ID NO.40: Amino acid sequence of hIFNα-HA-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEGGGGSGG
GGSGGGGSRTDTICIGYHANNSTDTVDTVLEKNVTVTHSVNLLEDSHNGKLCRLKGIA
PLQLGKCNIAGWLLGNPECDPLLPVRSWSYIVETPNSENGICYPGDFIDYEELREQLSSV
SSFERFEIFPKESSWPNHNTNGVTAACSHEGKSSFYRNLLWLTEKEGSYPKLKNSYVNK
KGKEVLVLWGIHHPPNSKEQQNLYQNENAYVSVVTSNYNRRFTPEIAERPKVRDQAG
RMNYYWTLLKPGDTIIFEANGNLIAPMYAFALSRGFGSGIITSNASMHECNTKCQTPLG
AINSSLPYQNIHPVTIGECPKYVRSAKLRMVTGLRNNPSIQSRFEDKTHTCPPCPAPELL
GGPSVFLFPPKPKDQLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFLYSKLT
VDKSRWQQGNVFSCSVLHEALHNHYTQKSLSLSPGK
   SEQ ID NO.41: Amino acid sequence of hIFNα-E7 (HPV)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEGGGGSGG
GGSGGGGSRTMHGDTPTLHEYMLDLQPETTDLYCYEQLNDSSEEEDEIDGPAGQAEPD
RAHYNIVTFCCKCDSTLRLCVQSTHVDIRTLEDLLMGTLGIVCPICSQKPFEDKTHTCPP
CPAPELLGGPSVFLFPPKPKDQLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGS
FLYSKLTVDKSRWQQGNVFSCSVLHEALHNHYTQKSLSLSPGK
   SEQ ID NO.42: Amino acid sequence of the first chain hIFN-Fc-hole in heterodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKESGGGGSG
GGGSGGGGSGGGGRTDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
 
CKVSNKALPAPIEKTISKAKGQPREPQVCTLPPSRDELTKNQVSLSCAVKGFYPSDIAVE
WESNGQPENNYKTTPPVLDSDGSFKLVSKLTVDKSRWQQGNVFSCSVMHEALHNHYT
QKSLSLSPGK
   SEQ ID NO.43: Amino acid sequence of the second chain Pres1-Fc-knob in heterodimer hIFNα-Pres1-Fc 
MGQNLSTSNPLGFFPDHQLDPAFRANTANPDWDFNPNKDTWPDANKVGAGAF
GLGFTPPHGGLLGWSPQAQGILQTLPANPPPASTNRQTGRQPTPLSPPLRNTHPQAFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVCTLPPSRDELTKNQVSLSCAVKGFYPSDIAVEWESNGQPENNYKTTPP
VLDSDGSFFLVSKLTVDKSRWQQGNVFSCSVMHEALHNHYTQKSLSLSPGK
   SEQ ID NO.44: Amino acid sequence of the second chain RBD (SARS-CoV-2)-Fc-knob in heterodimer hIFNα-RBD (SARA-CoV-2)-Fc 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNFFEDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPCRDELTKNQVSLWCLVKGFYPSDIAVEWESNGQPENNYKTTPPVLDSD
GSFFLYSKLTVDKSRWQQGNVFSCSVMHEALHNHYTQKSLSLSPGK
   SEQ ID NO.45: Amino acid sequence of the second chain HA-Fc-knob in heterodimer hIFNα-HA-Fc 
DTICIGYHANNSTDTVDTVLEKNVTVTHSVNLLEDSHNGKLCRLKGIAPLQLGK
CNIAGWLLGNPECDPLLPVRSWSYIVETPNSENGICYPGDFIDYEELREQLSSVSSFERFE
IFPKESSWPNHNTNGVTAACSHEGKSSFYRNLLWLTEKEGSYPKLKNSYVNKKGKEVL
VLWGIHHPPNSKEQQNLYQNENAYVSVVTSNYNRRFTPEIAERPKVRDQAGRMNYYW
TLLKPGDTIIFEANGNLIAPMYAFALSRGFGSGIITSNASMHECNTKCQTPLGAINSSLPY
QNIHPVTIGECPKYVRSAKLRMVTGLRNNPSIQSRFEDKTHTCPPCPAPELLGGPSVFLF
PPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPCRDEL
TKNQVSLWCLVKGFYPSDIAVEWESNGQPENNYKTTPPVLDSDGSFFLYSKLTVDKSR
WQQGNVFSCSVMHEALHNHYTQKSLSLSPGK
   SEQ ID NO.46: Amino acid sequence of the second chain E7(HPV)-Fc-knob in heterodimer hIFNα-E7 (HPV)-Fc 
MHGDTPTLHEYMLDLQPETTDLYCYEQLNDSSEEEDEIDGPAGQAEPDRAHYNI
VTFCCKCDSTLRLCVQSTHVDIRTLEDLLMGTLGIVCPICSQKPFEDKTHTCPPCPAPEL
 
LGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGK
5. Sequences of Human IFN and Pan epitope-containing vaccine IFNα-Pan-antigen-Fc sequence:
   SEQ ID NO.47: Amino acid sequence of hIFNα-Pan-Pres 1-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEAKFVAAW
TLKAAAGSGSGSMGQNLSTSNPLGFFPDHQLDPAFRANTANPDWDFNPNKDTWPDAN
KVGAGAFGLGFTPPHGGLLGWSPQAQGILQTLPANPPPASTNRQTGRQPTPLSPPLRNT
HPQAFEDKTHTCPPCPAPELLGGPSVFLFPPKPKDQLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQPEN
NYKTTPPVLDSDGSFLYSKLTVDKSRWQQGNVFSCSVLHEALHNHYTQKSLSLSPGK
   SEQ ID NO.48: Amino acid sequence of hIFNα-Pan-RBD (SARS-CoV-2)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEAKFVAAW
TLKAAAGSGSGSRVQPTESIVRFPNITNLCPFGEVFNATRFASVYAWNRKRISNCVADY
SVLYNSASFSTFKCYGVSPTKLNDLCFTNVYADSFVIRGDEVRQIAPGQTGKIADYNYK
LPDDFTGCVIAWNSNNLDSKVGGNYNYLYRLFRKSNLKPFERDISTEIYQAGSTPCNGV
EGFNCYFPLQSYGFQPTNGVGYQPYRVVVLSFELLHAPATVCGPKKSTNLVKNKCVNF
FEDKTHTCPPCPAPELLGGPSVFLFPPKPKDQLMISRTPEVTCVVVDVSHEDPEVKFNW
YVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKT
TPPVLDSDGSFLYSKLTVDKSRWQQGNVFSCSVLHEALHNHYTQKSLSLSPGK
   SEQ ID NO.49: Amino acid sequence of hIFNα-Pan-HA-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEAKFVAAW
TLKAAAGSGSGSDTICIGYHANNSTDTVDTVLEKNVTVTHSVNLLEDSHNGKLCRLKG
IAPLQLGKCNIAGWLLGNPECDPLLPVRSWSYIVETPNSENGICYPGDFIDYEELREQLS
SVSSFERFEIFPKESSWPNHNTNGVTAACSHEGKSSFYRNLLWLTEKEGSYPKLKNSYV
NKKGKEVLVLWGIHHPPNSKEQQNLYQNENAYVSVVTSNYNRRFTPEIAERPKVRDQ
 
AGRMNYYWTLLKPGDTIIFEANGNLIAPMYAFALSRGFGSGIITSNASMHECNTKCQTP
LGAINSSLPYQNIHPVTIGECPKYVRSAKLRMVTGLRNNPSIQSRFEDKTHTCPPCPAPE
LLGGPSVFLFPPKPKDQLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFLYSK
LTVDKSRWQQGNVFSCSVLHEALHNHYTQKSLSLSPGK
   SEQ ID NO.50: Amino acid sequence of hIFNα-Pan-E7 (HPV)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKEAKFVAAW
TLKAAAGSGSGSMHGDTPTLHEYMLDLQPETTDLYCYEQLNDSSEEEDEIDGPAGQAE
PDRAHYNIVTFCCKCDSTLRLCVQSTHVDIRTLEDLLMGTLGIVCPICSQKPFEDKTHTC
PPCPAPELLGGPSVFLFPPKPKDQLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFLYSKLTVDKSRWQQGNVFSCSVLHEALHNHYTQKSLSLSPGK
   SEQ ID NO.51: Amino acid sequence of the first chain hIFNα-Fc-hole in heterodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFQRITLYLKEKKYSPCAWEVVRAEIMRSFSLSTNLQESLRSKESGGGGSG
GGGSGGGGSGGGGRTDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYT
QKSLSLSPGK
   SEQ ID NO.52: Amino acid sequence of the second chain Pan-Pres1-Fc-knob in heterodimer hIFNα-Pan-Pres 1-Fc 
AKFVAAWTLKAAAGSGSGSMGQNLSTSNPLGFFPDHQLDPAFRANTANPDWD
FNPNKDTWPDANKVGAGAFGLGFTPPHGGLLGWSPQAQGILQTLPANPPPASTNRQTG
RQPTPLSPPLRNTHPQAFEDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKE
YKCKVSNKALPAPIEKTISKAKGQPREPQVYTLPPCRDELTKNQVSLWCLVKGFYPSDI
AVEWESNGQPENNYKTTPPVLDSDGSFFLYSKLTVDKSRWQQGNVFSCSVMHEALHN
HYTQKSLSLSPGK
   SEQ ID NO.53: Amino acid sequence of the second chain Pan-RBD (SARS-CoV-2)-Fc-knob in heterodimer hIFNα-Pan-RBD (SARA-CoV-2)-Fc 
AKFVAAWTLKAAAGSGSGSRVQPTESIVRFPNITNLCPFGEVFNATRFASVYAW
NRKRISNCVADYSVLYNSASFSTFKCYGVSPTKLNDLCFTNVYADSFVIRGDEVRQIAP
GQTGKIADYNYKLPDDFTGCVIAWNSNNLDSKVGGNYNYLYRLFRKSNLKPFERDIST
EIYQAGSTPCNGVEGFNCYFPLQSYGFQPTNGVGYQPYRVVVLSFELLHAPATVCGPK
KSTNLVKNKCVNFFEDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVYTLPPCRDELTKNQVSLWCLVKGFYPSDIAV
EWESNGQPENNYKTTPPVLDSDGSFFLYSKLTVDKSRWQQGNVFSCSVMHEALHNHY
TQKSLSLSPGK
   SEQ ID NO.54: Amino acid sequence of the second chain Pan-HA-Fc-knob in heterodimer hIFNα-Pan-HA-Fc 
AKFVAAWTLKAAAGSGSGSDTICIGYHANNSTDTVDTVLEKNVTVTHSVNLLE
DSHNGKLCRLKGIAPLQLGKCNIAGWLLGNPECDPLLPVRSWSYIVETPNSENGICYPG
DFIDYEELREQLSSVSSFERFEIFPKESSWPNHNTNGVTAACSHEGKSSFYRNLLWLTEK
EGSYPKLKNSYVNKKGKEVLVLWGIHHPPNSKEQQNLYQNENAYVSVVTSNYNRRFT
PEIAERPKVRDQAGRMNYYWTLLKPGDTIIFEANGNLIAPMYAFALSRGFGSGIITSNAS
MHECNTKCQTPLGAINSSLPYQNIHPVTIGECPKYVRSAKLRMVTGLRNNPSIQSRFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK
   SEQ ID NO.55: Amino acid sequence of the second chain Pan-HA-Fc-knob in heterodimer hIFNα-Pan-E7(HPV)-Fc 
AKFVAAWTLKAAAGSGSGSMHGDTPTLHEYMLDLQPETTDLYCYEQLNDSSE
EEDEIDGPAGQAEPDRAHYNIVTFCCKCDSTLRLCVQSTHVDIRTLEDLLMGTLGIVCPI
CSQKPFEDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPCRDELTKNQVSLWCLVKGFYPSDIAVEWESNGQPE
NNYKTTPPVLDSDGSFFLYSKLTVDKSRWQQGNVFSCSVMHEALHNHYTQKSLSLSPG
K
6. Sequences of Human mutated IFN vaccine hmIFNα-Pan-antigen-Fc:
   SEQ ID NO.56: Amino acid sequence of hmIFNα-Pres1-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEGGGGSGG
GGSGGGGSRTMGQNLSTSNPLGFFPDHQLDPAFRANTANPDWDFNPNKDTWPDANK
VGAGAFGLGFTPPHGGLLGWSPQAQGILQTLPANPPPASTNRQTGRQPTPLSPPLRNTH
 
PQAFEDKTHTCPPCPAPELLGGPSVFLFPPKPKDQLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENN
YKTTPPVLDSDGSFLYSKLTVDKSRWQQGNVFSCSVLHEALHNHYTQKSLSLSPGK
 SEQ ID NO.57: Amino acid sequence of hmIFNα-RBD (SARS-CoV-2)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEGGGGSGG
GGSGGGGSRTRVQPTESIVRFPNITNLCPFGEVFNATRFASVYAWNRKRISNCVADYSV
LYNSASFSTFKCYGVSPTKLNDLCFTNVYADSFVIRGDEVRQIAPGQTGKIADYNYKLP
DDFTGCVIAWNSNNLDSKVGGNYNYLYRLFRKSNLKPFERDISTEIYQAGSTPCNGVE
GFNCYFPLQSYGFQPTNGVGYQPYRVVVLSFELLHAPATVCGPKKSTNLVKNKCVNFF
EDKTHTCPPCPAPELLGGPSVFLFPPKPKDQLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKTT
PPVLDSDGSFLYSKLTVDKSRWQQGNVFSCSVLHEALHNHYTQKSLSLSPGK
   SEQ ID NO.58: Amino acid sequence of hmIFNα-HA-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEGGGGSGG
GGSGGGGSRTDTICIGYHANNSTDTVDTVLEKNVTVTHSVNLLEDSHNGKLCRLKGIA
PLQLGKCNIAGWLLGNPECDPLLPVRSWSYIVETPNSENGICYPGDFIDYEELREQLSSV
SSFERFEIFPKESSWPNHNTNGVTAACSHEGKSSFYRNLLWLTEKEGSYPKLKNSYVNK
KGKEVLVLWGIHHPPNSKEQQNLYQNENAYVSVVTSNYNRRFTPEIAERPKVRDQAG
RMNYYWTLLKPGDTIIFEANGNLIAPMYAFALSRGFGSGIITSNASMHECNTKCQTPLG
AINSSLPYQNIHPVTIGECPKYVRSAKLRMVTGLRNNPSIQSRFEDKTHTCPPCPAPELL
GGPSVFLFPPKPKDQLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFLYSKLT
VDKSRWQQGNVFSCSVLHEALHNHYTQKSLSLSPGK
   SEQ ID NO.59: Amino acid sequence of hmIFNα-E7 (HPV)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEGGGGSGG
GGSGGGGSRTMHGDTPTLHEYMLDLQPETTDLYCYEQLNDSSEEEDEIDGPAGQAEPD
RAHYNIVTFCCKCDSTLRLCVQSTHVDIRTLEDLLMGTLGIVCPICSQKPFEDKTHTCPP
 
CPAPELLGGPSVFLFPPKPKDQLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGS
FLYSKLTVDKSRWQQGNVFSCSVLHEALHNHYTQKSLSLSPGK
   SEQ ID NO.60: Amino acid sequence of the first chain hmIFN-Fc-hole in heterodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKESGGGGSG
GGGSGGGGSGGGGRTDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYT
QKSLSLSPGK
   SEQ ID NO: 61: Amino acid sequence of the second chain Pres1-Fc-knob in heterodimer hmIFNα-Pres1-Fc 
MGQNLSTSNPLGFFPDHQLDPAFRANTANPDWDFNPNKDTWPDANKVGAGAF
GLGFTPPHGGLLGWSPQAQGILQTLPANPPPASTNRQTGRQPTPLSPPLRNTHPQAFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK
   SEQ ID NO.62: Amino acid sequence of the second chain RBD (SARS-CoV-2)-Fc-knob in heterodimer hmIFNα-RBD (SARA-CoV-2)-Fc 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNFFEDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQP
REPQVYTLPPCRDELTKNQVSLWCLVKGFYPSDIAVEWESNGQPENNYKTTPPVLDSD
GSFFLYSKLTVDKSRWQQGNVFSCSVMHEALHNHYTQKSLSLSPGK
   SEQ ID NO.63: Amino acid sequence of the second chain HA-Fc-knob in heterodimer hmIFNα-HA-Fc 
DTICIGYHANNSTDTVDTVLEKNVTVTHSVNLLEDSHNGKLCRLKGIAPLQLGK
CNIAGWLLGNPECDPLLPVRSWSYIVETPNSENGICYPGDFIDYEELREQLSSVSSFERFE
IFPKESSWPNHNTNGVTAACSHEGKSSFYRNLLWLTEKEGSYPKLKNSYVNKKGKEVL
VLWGIHHPPNSKEQQNLYQNENAYVSVVTSNYNRRFTPEIAERPKVRDQAGRMNYYW
 
TLLKPGDTIIFEANGNLIAPMYAFALSRGFGSGIITSNASMHECNTKCQTPLGAINSSLPY
QNIHPVTIGECPKYVRSAKLRMVTGLRNNPSIQSRFEDKTHTCPPCPAPELLGGPSVFLF
PPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPCRDEL
TKNQVSLWCLVKGFYPSDIAVEWESNGQPENNYKTTPPVLDSDGSFFLYSKLTVDKSR
WQQGNVFSCSVMHEALHNHYTQKSLSLSPGK
   SEQ ID NO.64: Amino acid sequence of the second chain HA-Fc-knob in heterodimer hmIFNα-E7 (HPV)-Fc 
MHGDTPTLHEYMLDLQPETTDLYCYEQLNDSSEEEDEIDGPAGQAEPDRAHYNI
VTFCCKCDSTLRLCVQSTHVDIRTLEDLLMGTLGIVCPICSQKPFEDKTHTCPPCPAPEL
LGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGK
7. Sequences of Human mutated IFN and Pan epitope-containing vaccine hmIFNα-Pan epitope-antigen-Fc:
   SEQ ID NO.65: Amino acid sequence of hmIFNα-Pan-Pres 1-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEAKFVAAW
TLKAAAGSGSGSMGQNLSTSNPLGFFPDHQLDPAFRANTANPDWDFNPNKDTWPDAN
KVGAGAFGLGFTPPHGGLLGWSPQAQGILQTLPANPPPASTNRQTGRQPTPLSPPLRNT
HPQAFEDKTHTCPPCPAPELLGGPSVFLFPPKPKDQLMISRTPEVTCVVVDVSHEDPEV
KFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQPEN
NYKTTPPVLDSDGSFLYSKLTVDKSRWQQGNVFSCSVLHEALHNHYTQKSLSLSPGK
   SEQ ID NO.66: Amino acid sequence of hmIFNα-Pan -RBD (SARS-CoV-2)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEAKFVAAW
TLKAAAGSGSGSRVQPTESIVRFPNITNLCPFGEVFNATRFASVYAWNRKRISNCVADY
SVLYNSASFSTFKCYGVSPTKLNDLCFTNVYADSFVIRGDEVRQIAPGQTGKIADYNYK
LPDDFTGCVIAWNSNNLDSKVGGNYNYLYRLFRKSNLKPFERDISTEIYQAGSTPCNGV
EGFNCYFPLQSYGFQPTNGVGYQPYRVVVLSFELLHAPATVCGPKKSTNLVKNKCVNF
FEDKTHTCPPCPAPELLGGPSVFLFPPKPKDQLMISRTPEVTCVVVDVSHEDPEVKFNW
YVDGVEVHNAKTKPREEQYNSTYRVVSVLTVLHQDWLNGKEYKCKVSNKALPAPIEK
 
TISKAKGQPREPQVYTLPPSRDELTKNQVSLTCLVKGFYPSDIAVEWESNGQPENNYKT
TPPVLDSDGSFLYSKLTVDKSRWQQGNVFSCSVLHEALHNHYTQKSLSLSPGK
   SEQ ID NO.67: Amino acid sequence of hmIFNα-Pan-HA-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEAKFVAAW
TLKAAAGSGSGSDTICIGYHANNSTDTVDTVLEKNVTVTHSVNLLEDSHNGKLCRLKG
IAPLQLGKCNIAGWLLGNPECDPLLPVRSWSYIVETPNSENGICYPGDFIDYEELREQLS
SVSSFERFEIFPKESSWPNHNTNGVTAACSHEGKSSFYRNLLWLTEKEGSYPKLKNSYV
NKKGKEVLVLWGIHHPPNSKEQQNLYQNENAYVSVVTSNYNRRFTPEIAERPKVRDQ
AGRMNYYWTLLKPGDTIIFEANGNLIAPMYAFALSRGFGSGIITSNASMHECNTKCQTP
LGAINSSLPYQNIHPVTIGECPKYVRSAKLRMVTGLRNNPSIQSRFEDKTHTCPPCPAPE
LLGGPSVFLFPPKPKDQLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFLYSK
LTVDKSRWQQGNVFSCSVLHEALHNHYTQKSLSLSPGK
   SEQ ID NO.68: Amino acid sequence of hmIFNα-Pan-E7 (HPV)-Fc in homodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKEAKFVAAW
TLKAAAGSGSGSMHGDTPTLHEYMLDLQPETTDLYCYEQLNDSSEEEDEIDGPAGQAE
PDRAHYNIVTFCCKCDSTLRLCVQSTHVDIRTLEDLLMGTLGIVCPICSQKPFEDKTHTC
PPCPAPELLGGPSVFLFPPKPKDQLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDS
DGSFLYSKLTVDKSRWQQGNVFSCSVLHEALHNHYTQKSLSLSPGK
   SEQ ID NO.69: Amino acid sequence of the first chain hmIFNα4-Fc-hole in heterodimer 
CDLPQTHSLGSRRTLMLLAQMRRISLFSCLKDRHDFGFPQEEFGNQFQKAETIPV
LHEMIQQIFNLFSTKDSSAAWDETLLDKFYTELYQQLNDLEACVIQGVGVTETPLMKE
DSILAVRKYFRRITLYLKEKKYSPCAWEVVRAEIMRSFSLSTNLQESLRSKESGGGGSG
GGGSGGGGSGGGGRTDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALHNHYT
QKSLSLSPGK
   SEQ ID NO.70: Amino acid sequence of the second chain Pan-Pres1-Fc-knob in heterodimer hmIFNα-Pan-Pres1-Fc 
AKFVAAWTLKAAAGSGSGSMGQNLSTSNPLGFFPDHQLDPAFRANTANPDWD
FNPNKDTWPDANKVGAGAFGLGFTPPHGGLLGWSPQAQGILQTLPANPPPASTNRQTG
RQPTPLSPPLRNTHPQAFEDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKE
YKCKVSNKALPAPIEKTISKAKGQPREPQVYTLPPCRDELTKNQVSLWCLVKGFYPSDI
AVEWESNGQPENNYKTTPPVLDSDGSFFLYSKLTVDKSRWQQGNVFSCSVMHEALHN
HYTQKSLSLSPGK
   SEQ ID NO.71: Amino acid sequence of the second chain Pan-RBD (SARS-CoV-2)-Fc-knob in heterodimer hmIFNα-Pan-RBD (SARA-CoV-2)-Fc 
AKFVAAWTLKAAAGSGSGSRVQPTESIVRFPNITNLCPFGEVFNATRFASVYAW
NRKRISNCVADYSVLYNSASFSTFKCYGVSPTKLNDLCFTNVYADSFVIRGDEVRQIAP
GQTGKIADYNYKLPDDFTGCVIAWNSNNLDSKVGGNYNYLYRLFRKSNLKPFERDIST
EIYQAGSTPCNGVEGFNCYFPLQSYGFQPTNGVGYQPYRVVVLSFELLHAPATVCGPK
KSTNLVKNKCVNFFEDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYK
CKVSNKALPAPIEKTISKAKGQPREPQVYTLPPCRDELTKNQVSLWCLVKGFYPSDIAV
EWESNGQPENNYKTTPPVLDSDGSFFLYSKLTVDKSRWQQGNVFSCSVMHEALHNHY
TQKSLSLSPGK
   SEQ ID NO.72: Amino acid sequence of the second chain Pan-HA-Fc-knob in heterodimer hmIFNα-Pan-HA-Fc 
AKFVAAWTLKAAAGSGSGSDTICIGYHANNSTDTVDTVLEKNVTVTHSVNLLE
DSHNGKLCRLKGIAPLQLGKCNIAGWLLGNPECDPLLPVRSWSYIVETPNSENGICYPG
DFIDYEELREQLSSVSSFERFEIFPKESSWPNHNTNGVTAACSHEGKSSFYRNLLWLTEK
EGSYPKLKNSYVNKKGKEVLVLWGIHHPPNSKEQQNLYQNENAYVSVVTSNYNRRFT
PEIAERPKVRDQAGRMNYYWTLLKPGDTIIFEANGNLIAPMYAFALSRGFGSGIITSNAS
MHECNTKCQTPLGAINSSLPYQNIHPVTIGECPKYVRSAKLRMVTGLRNNPSIQSRFED
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK
   SEQ ID NO.73: Amino acid sequence of the second chain Pan-HA-Fc-knob in heterodimer hmIFNα-Pan-E7 (HPV)-Fc 
AKFVAAWTLKAAAGSGSGSMHGDTPTLHEYMLDLQPETTDLYCYEQLNDSSE
EEDEIDGPAGQAEPDRAHYNIVTFCCKCDSTLRLCVQSTHVDIRTLEDLLMGTLGIVCPI
CSQKPFEDKTHTCPPCPAPELLGGPSVFLFPPKPKDTLMISRTPEVTCVVVDVSHEDPEV
 
KFNWYVDGVEVHNAKTKPREEQYNSTYRVVSVLTVLHQDWLNGKEYKCKVSNKALP
APIEKTISKAKGQPREPQVYTLPPCRDELTKNQVSLWCLVKGFYPSDIAVEWESNGQPE
NNYKTTPPVLDSDGSFFLYSKLTVDKSRWQQGNVFSCSVMHEALHNHYTQKSLSLSPG
K
8. Sequences of antibodies used to replace the Fc
   SEQ ID NO.20: Amino acid sequence of ScFv (PD-L1) 
DIQMTQSPSSLSASVGDRVTITCRASQDVSTAVAWYQQKPGKAPKLLIYSASFL
YSGVPSRFSGSGSGTDFTLTISSLQPEDFATYYCQQYLYHPATFGQGTKVEIKRGGGGS
GGGGSGGGGSEVQLVESGGGLVQPGGSLRLSCAASGFTFSDSWIHWVRQAPGKGLEW
VAWISPYGGSTYYADSVKGRFTISADTSKNTAYLQMNSLRAEDTAVYYCARRHWPGG
FDYWGQGTLVTVSA
   SEQ ID NO.74: Amino acid sequence of Anti-PD-L1 VH 
EVQLVESGGGLVQPGGSLRLSCAASGFTFSDSWIHWVRQAPGKGLEWVAWISP
YGGSTYYADSVKGRFTISADTSKNTAYLQMNSLRAEDTAVYYCARRHWPGGFDYWG
QGTLVTVSA
   SEQ ID NO.75: Amino acid sequence of Anti-PD-L1 VL 
DIQMTQSPSSLSASVGDRVTITCRASQDVSTAVAWYQQKPGKAPKLLIYSASFL
YSGVPSRFSGSGSGTDFTLTISSLQPEDFATYYCQQYLYHPATFGQGTKVEIKR
9. Other virus antigen sequences
   SEQ ID NO.76: Amino acid sequence of SARS-CoV-2 Spike protein 
VNLTTRTQLPPAYTNSFTRGVYYPDKVFRSSVLHSTQDLFLPFFSNVTWFHAIH
VSGTNGTKRFDNPVLPFNDGVYFASTEKSNIIRGWIFGTTLDSKTQSLLIVNNATNVVIK
VCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRS
YLTPGDSSSGWTAGAAAYYVGYLQPRTFLLKYNENGTITDAVDCALDPLSETKCTLKS
FTVEKGIYQTSNFRVQPTESIVRFPNITNLCPFGEVFNATRFASVYAWNRKRISNCVADY
SVLYNSASFSTFKCYGVSPTKLNDLCFTNVYADSFVIRGDEVRQIAPGQTGKIADYNYK
LPDDFTGCVIAWNSNNLDSKVGGNYNYLYRLFRKSNLKPFERDISTEIYQAGSTPCNGV
EGFNCYFPLQSYGFQPTNGVGYQPYRVVVLSFELLHAPATVCGPKKSTNLVKNKCVNF
NFNGLTGTGVLTESNKKFLPFQQFGRDIADTTDAVRDPQTLEILDITPCSFGGVSVITPG
TNTSNQVAVLYQDVNCTEVPVAIHADQLTPTWRVYSTGSNVFQTRAGCLIGAEHVNN
SYECDIPIGAGICASYQTQTNSPRRARSVASQSIIAYTMSLGAENSVAYSNNSIAIPTNFTI
SVTTEILPVSMTKTSVDCTMYICGDSTECSNLLLQYGSFCTQLNRALTGIAVEQDKNTQ
EVFAQVKQIYKTPPIKDFGGFNFSQILPDPSKPSKRSFIEDLLFNKVTLADAGFIKQYGDC
LGDIAARDLICAQKFNGLTVLPPLLTDEMIAQYTSALLAGTITSGWTFGAGAALQIPFA
MQMAYRFNGIGVTQNVLYENQKLIANQFNSAIGKIQDSLSSTASALGKLQD
VVNQNAQALNTLVKQLSSNFGAISSVLNDILSRLDKVEAEVQIDRLITGRLQSLQTYVT
 
QQLIRAAEIRASANLAATKMSECVLGQSKRVDFCGKGYHLMSFPQSAPHGVVFLHVTY
VPAQEKNFTTAPAICHDGKAHFPREGVFVSNGTHWFVTQRNFYEPQIITTDNTFVSGNC
DVVIGIVNNTVYDPLQPELDSFKEELDKYFKNHTSPDVDLGDISGINASVVNIQKEIDRL
NEVAKNLNESLIDLQELGKYEQYIKWPWYIWLGFIAGLIAIVMVTIMLCCMTSCCSCLK
GCCSCGSCCKFDEDDSEPVLKGVKLHYT
   SEQ ID NO. 77: Amino acid sequence of SARS-CoV-2 S1 protein 
VNLTTRTQLPPAYTNSFTRGVYYPDKVFRSSVLHSTQDLFLPFFSNVTWFHAIH
VSGTNGTKRFDNPVLPFNDGVYFASTEKSNIIRGWIFGTTLDSKTQSLLIVNNATNVVIK
VCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRS
YLTPGDSSSGWTAGAAAYYVGYLQPRTFLLKYNENGTITDAVDCALDPLSETKCTLKS
FTVEKGIYQTSNFRVQPTESIVRFPNITNLCPFGEVFNATRFASVYAWNRKRISNCVADY
SVLYNSASFSTFKCYGVSPTKLNDLCFTNVYADSFVIRGDEVRQIAPGQTGKIADYNYK
LPDDFTGCVIAWNSNNLDSKVGGNYNYLYRLFRKSNLKPFERDISTEIYQAGSTPCNGV
EGFNCYFPLQSYGFQPTNGVGYQPYRVVVLSFELLHAPATVCGPKKSTNLVKNKCVNF
NFNGLTGTGVLTESNKKFLPFQQFGRDIADTTDAVRDPQTLEILDITPCSFGGVSVITPG
TNTSNQVAVLYQDVNCTEVPVAIHADQLTPTWRVYSTGSNVFQTRAGCLIGAEHVNN
SYECDIPIGAGICASYQTQTNSPRRAR
   SEQ ID NO. 78: Amino acid sequence of SARS-CoV-2 original strain RBD protein 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNF
   SEQ ID NO. 79: Amino acid sequence of RBD protein of SARS-CoV-2 British mutant strain (B.1.1.7, Alpha) 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFP
LQSYGFQPTYGVGYQPYRVVVLSFELLHAPATVCGPKKSTNLVKNKCVNF
   SEQ ID NO. 80: Amino acid sequence of RBD protein of SARS-CoV-2 South Africa mutant strain (B.1.351, Beta) 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGNIADYNYKLPDDFTGC
VIAWNSNNLDSKVGGNYNYLYRLFRKSNLKPFERDISTEIYQAGSTPCNGVKGFNCYFP
LQSYGFQPTYGVGYQPYRVVVLSFELLHAPATVCGPKKSTNLVKNKCVNF
   SEQ ID NO. 81: Amino acid sequence of RBD protein of SARS-CoV-2 Brazil mutant strain (P.1) 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGTIADYNYKLPDDFTGC
VIAWNSNNLDSKVGGNYNYLYRLFRKSNLKPFERDISTEIYQAGSTPCNGVKGFNCYFP
LQSYGFQPTYGVGYQPYRVVVLSFELLHAPATVCGPKKSTNLVKNKCVNF
   SEQ ID NO. 82: Amino acid sequence of RBD protein of SARS-CoV-2 California mutant strain (B.1.429) 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSTPCNGVEGFNCYFP
LQSYGFQPTNGVGYQPYRVVVLSFELLHAPATVCGPKKSTNLVKNKCVNF
   SEQ ID NO. 83: Amino acid sequence of RBD protein of SARS-CoV-2 India B.1.617, B.1.617.1 (Kappa), and B.1.617.3 mutant strains (B.1.429) 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSTPCNGVQGFNCYF
PLQSYGFQPTNGVGYQPYRVVVLSFELLHAPATVCGPKKSTNLVKNKCVNF
   SEQ ID NO. 84: Amino acid sequence of RBD protein of SARS-CoV-2 India second generation B.1.617.2 (Delta) mutant strain 
RVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGC
VIAWNSNNLDSKVGGNYNYRYRLFRKSNLKPFERDISTEIYQAGSKPCNGVEGFNCYF
PLQSYGFQPTNGVGYQPYRVVVLSFELLHAPATVCGPKKSTNLVKNKCVNF
10. Other tumor antigen sequences:
   Amino acid sequences of murine Her2 extracellular domains II, III and IV involved in the Examples:
      SEQ ID NO. 85 Mouse Her2-extracellular domain 2: 
SRACPPCAPACKDNHCWGESPEDCQILTGTICTSGCARCKGRLPTDCCHEQCAA
GCTGPKHSDCLACLHFNHSGICELHCPALVTYNTDTFESMHNPEGRYTFGASCVTTCP
YNYLSTEVGSCTLVCPPNNQEVTAEDGTQRCEKCSKPC
      SEQ ID NO. 86 Mouse Her2-extracellular domain 3: 
GCKKIFGSLAFLPESFDGDPSSGIAPLRPEQLQVFETLEEITGYLYISAWPDSLRDL
SVFQNLRIIRGRILHDGAYSLTLQGLGIHSLGLRSLRELGSGLALIHRNAHLCFVHTVPW
DQLF
      SEQ ID NO. 87 Mouse Her2-extracellular domain 4: 
VCNSLCAHGHCWGPGPTQCVNCSHFLRGQECVEECRVWKGLPREYVSDKRCL
PCHPECQPQNSSETCFGSEADQCAACAHYKDSSSCVARCPSGVKPDLSYMPIWKYPDE
EGICQPCPINCTHSCVDLDERGCP
   Amino acid sequences of human Her2 extracellular domains II, III and IV involved in the Examples:
      SEQ ID NO. 88 Human Her2-extracellular domain 2: 
SRACHPCSPMCKGSRCWGESSEDCQSLTRTVCAGGCARCKGPLPTDCCHEQCA
AGCTGPKHSDCLACLHFNHSGICELHCPALVTYNTDTFESMPNPEGRYTFGASCVTACP
YNYLSTDVGSCTLVCPLHNQEVTAEDGTQRCEKCSKPC
      SEQ ID NO. 89 Human Her2-extracellular domain 3: 
GCKKIFGSLAFLPESFDGDPASNTAPLQPEQLQVFETLEEITGYLYISAWPDSLPD
LSVFQNLQVIRGRILHNGAYSLTLQGLGISWLGLRSLRELGSGLALIHHNTHLCFVHTVP
W
      SEQ ID NO. 90 Human Her2-extracellular domain 4: 
CHQLCARGHCWGPGPTQCVNCSQFLRGQECVEECRVLQGLPREYVNARHCLP
CHPECQPQNGSVTCFGPEADQCVACAHYKDPPFCVARCPSGVKPDLSYMPIWKFPDEE
GACQPCPINCTHSCVDLDDKGCP
11. Herpes virus antigen sequences involved in the Examples:
   SEQ ID NO. 91 VZV Envelope glycoprotein E (aa 31-538) 
SVLRYDDFHTDEDKLDTNSVYEPYYHSDHAESSWVNRGESSRKAYDHNSPYIW
PRNDYDGFLENAHEHHGVYNQGRGIDSGERLMQPTQMSAQEDLGDDTGIHVIPTLNG
DDRHKIVNVDQRQYGDVFKGDLNPKPQGQRLIEVSVEENHPFTLRAPIQRIYGVRYTET
WSFLPSLTCTGDAAPAIQHICLKHTTCFQDVVVDVDCAENTKEDQLAEISYRFQGKKE
ADQPWIVVNTSTLFDELELDPPEIEPGVLKVLRTEKQYLGVYIWNMRGSDGTSTYATFL
VTWKGDEKTRNPTPAVTPQPRGAEFHMWNYHSHVFSVGDTFSLAMHLQYKIHEAPFD
LLLEWLYVPIDPTCQPMRLYSTCLYHPNAPQCLSHMNSGCTFTSPHLAQRVASTVYQN
CEHADNYTAYCLGISHMEPSFGLILHDGGTTLKFVDTPESLSGLYVFVVYFNGHVEAV
AYTVVSTVDHFVNAIEERGFPPTAGQPPATTKPKEITPVNPGTSPLLRY
   SEQ ID NO. 92 EBV Envelope glycoprotein GP350 (aa 1-425) 
MEAALLVCQYTIQSLIHLTGEDPGFFNVEIPEFPFYPTCNVCTADVNVTINFDVG
GKKHQLDLDFGQLTPHTKAVYQPRGAFGGSENATNLFLLELLGAGELALTMRSKKLPI
NVTTGEEQQVSLESVDVYFQDVFGTMWCHHAEMQNPVYLIPETVPYIKWDNCNSTNI
TAVVRAQGLDVTLPLSLPTSAQDSNFSVKTQMLGNEIDIECIMEDGEISQVLPGDNKFNI
TCSGYESHVPSGGILTSTSPVVTPIPGTGYAYSLRLTPRPVSRFLGNNSILYVFYSGNGPK
ASGGDYCIQSNIVFSDEIPASQDMPTNTTDITYVGDNATYSVPMVTSEDANSPNVTVTA
 
FWAWPNNTETDFKCKWTLTSGTPSGCENISGAFASNRTFDITVSGLGTAPKTLIITRTAT
NATTTTHKVIFSKAP
   SEQ ID NO. 93 HSV-2 Envelope glycoprotein gD (aa 26-339)

KYALADPSLKMADPNRFRGKNLPVLDRLTDPPGVKRVYHIQPSLEDPFQPPSIPI
TVYYAVLERACRSVLLHAPSEAPQIVRGASDEARKHTYNLTIAWYRMGDNCAIPITVM
EYTECPYNKSLGVCPIRTQPRWSYYDSFSAVSEDNLGFLMHAPAFETAGTYLRLVKIND
WTEITQFILEHRARASCKYALPLRIPPAACLTSKAYQQGVTVDSIGMLPRFIPENQRTVA
LYSLKIAGWHGPKPPYTSTLLPPELSDTTNATQPELVPEDPEDSALLEDPAGTVSSQIPP
NWHIPSIQDVAPHHAPAAPSNP

### BRIEF DESCRIPTION OF THE DRAWINGS

Figure 1 was a schematic diagram of the vaccine platform in the form of homodimer, arranged in the order of interferon-linking fragment 1-target antigen-immunoglobulin Fc (or antibody);
Figure 2 was a schematic diagram of the vaccine platform in the form of heterodimer, according to the combination of interferon-linking fragment 1-IgG1-hole and target antigen-IgG1-knob (or antibody);
Figure 3 was a schematic diagram of the vaccine platform in the form of heterodimer, according to the combination of interferon-linking fragment 1-IgG1-knob and target protein-IgG1-hole (or antibody);
Figure 4 was a schematic diagram of the vaccine platform in the form of homodimer, arranged in the order of interferon-linking fragment 1-Th cell helper epitope-linking fragment 2-target antigen-immunoglobulin Fc (or antibody);
Figure 5 was a schematic diagram of the vaccine platform in the form of heterodimer, according to the combination of interferon-linking fragment 1-IgG1-hole and Th cell helper epitope-linking fragment 2-target antigen-IgG1-knob (or antibody) ;
Figure 6 was a schematic diagram of the vaccine platform in the form of heterodimer, according to the combination of interferon-linking fragment 1-IgG1-knob and Th cell helper epitope-linking fragment 2-target antigen-IgG1-hole (or antibody).
Figure 7 showed the non-denatured protein SDS-PAGE electrophoresis map of Pres 1-Fc, and IFN-Pres1-Fc.

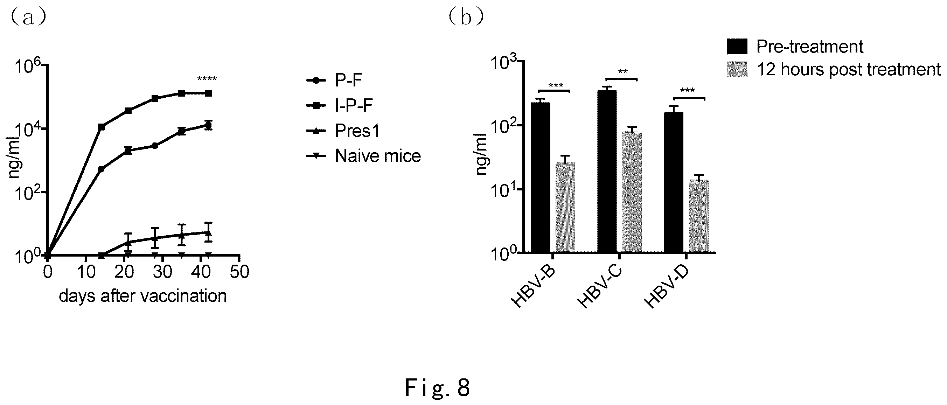

Figure 8 showed that compared with free preS1, the fusion proteins preS1-Fc and IFN-preS1-Fc could significantly enhance the immunity of antigen molecules and promote the production of broad-spectrum neutralizing antibodies. (a) C57/BL6 (n=8/group) mice were subcutaneously immunized with free hepatitis B Pres1, Pres1-Fc, and IFNα-Pres1-Fc proteins, and the level of Pres1-specific antibody in serum was detected by ELISA at specified time. (b) Mice (n=4) stably carrying three HBV genotypes were injected intravenously with serum from mice immunized with IFNα-Pres1-Fc protein, and the changes of Pres1 antigen in serum were detected 12 hours later.

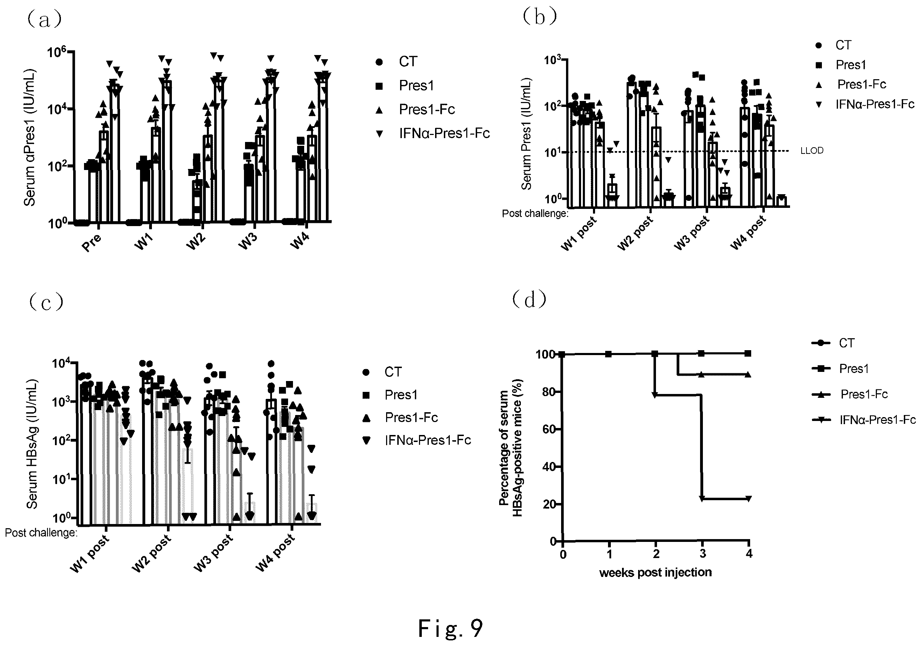

Figure 9 showed that IFNα-Pres1-Fc could be used as a prophylactic vaccine against hepatitis B. C57/BL6 mice were subcutaneously immunized with free hepatitis B Pres1, Pres1-Fc, and IFNα-Pres1-Fc proteins, and infected with 1x10¹¹ µg of AAV-HBV1.3 virus by tail vein at day 28 after inoculation. (a) Serum Anti-Pres1 levels before virus inoculation and at 1, 2, 3, and 4 weeks after virus inoculation. (b) Serum levels of Pres 1 detected at the indicated time points. (c) Serum HBsAg levels detected at weeks 1, 2, 3, and 4 by ELISA. (d) Proportion of HBsAg-positive mice after AAV-HBV1.3 virus inoculation.
Figure 10 showed IFNα-Pres1-Fc as a therapeutic vaccine for chronic B infection. C57/BL6 mice were infected with 1x10¹¹ µg of AAV-HBV1.3 virus by tail vein injection. After 6 weeks of infection, stable infected mice were selected (n=8/group), and subcutaneously inoculated with recombinant Pres1, IFNα-Pres1-Fc proteins once every two weeks for a total of three times. (a) Detection of Anti-Pres1 antigen in serum; (b) Detection of Pres1 antigen in serum; (c) Detection of HBV-related antigen HBsAg in mouse serum

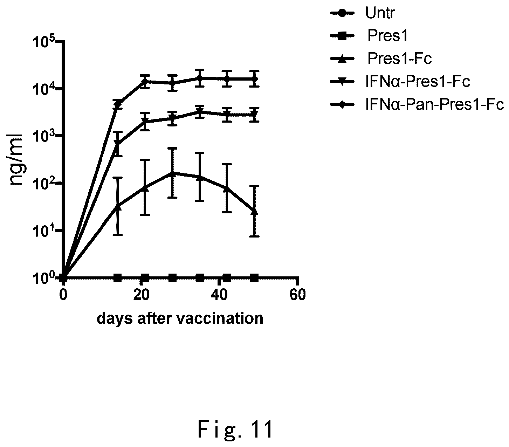

Figure 11 showed that Th cell helper epitopes enhanced the antibody response of IFNα-Pres1-Fc vaccine
   Compared with IFN-preS1-Fc, the IFN-Pan-preS1-Fc could significantly enhance the immunogenicity of antigen molecules. C57/BL6 (n=8/group) mice were subcutaneously immunized with hepatitis B Pres1, Pres1-Fc, and IFNα-Pres1-Fc proteins without aluminum adjuvant, and the level of Pres 1-specific antibody in serum was detected by ELISA at specified time.

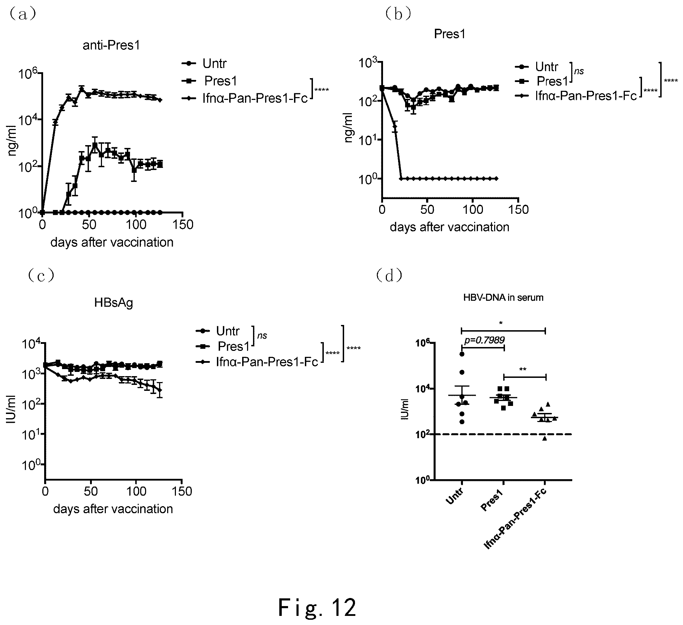

Figure 12 showed IFNα-Pan-Pres1-Fc as a therapeutic vaccine for chronic B infection. C57/BL6 mice were infected with 1x10¹¹ µg of AAV-HBV1.3 virus by tail vein injection. After 6 weeks of infection, stable infected mice were selected (n=8/group), and subcutaneously inoculated with recombinant Pres1, IFNα-Pres1-Fc proteins once every two weeks for a total of three times. (a) Detection of Anti-Pres1 antigen in serum; (b) Detection of Pres1 antigen in serum; (c) Detection of HBV-related antigen HBsAg level in mouse serum; (d) Detection of HBV-DNA level in mouse serum by QPCR.

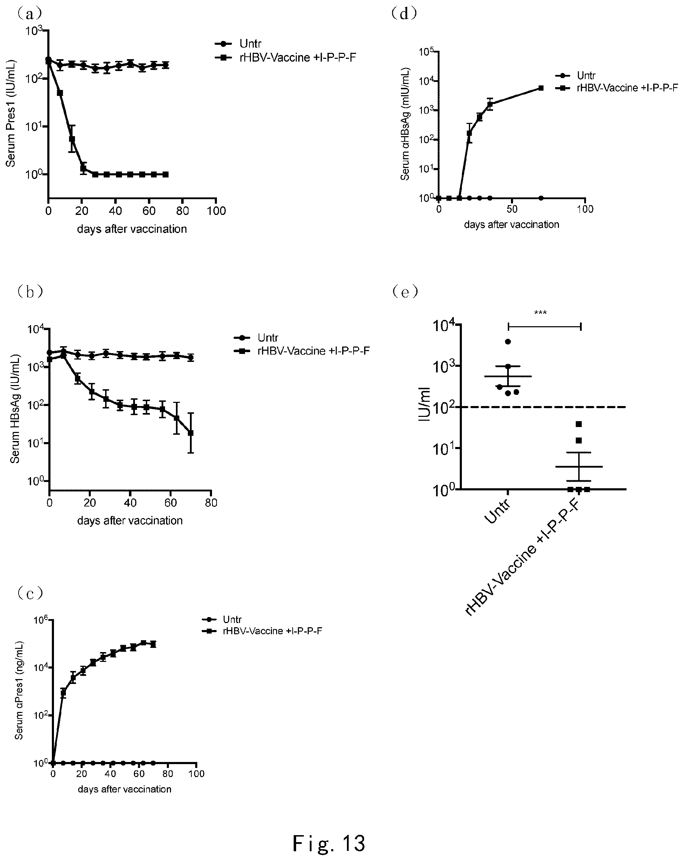

Figure 13 showed the combination of IFNα-Pres1-Fc and HBsAg commercial vaccine broke immune tolerance against HBsAg and induced HBsAg-HBsAb serological conversion. HBV Carrier mice were subcutaneously immunized with IFNα-Pres1-Fc and HBsAg commercial vaccine once every two weeks for a total of three times. (a) The level of Pres1 in the serum of HBV Carrier mice, (b) the level of HBsAg, (c) the level of Anti-Pres1 in serum, (d) the level of Anti-HBsAg in serum, (e) the level of HBV-DNA in serum. *** , p<0.001

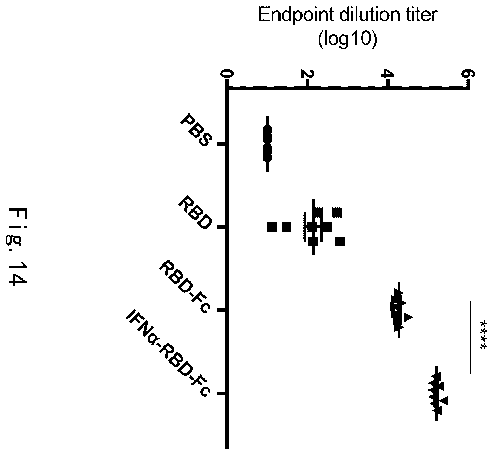

Figure 14 showed that the IFNα-RBD(SARS-CoV2)-Fc could cause a stronger antibody response than free SARS-Cov2 RBD protein. Balb/c (n=8/group) mice were inoculated with free SARS-Cov-2 RBD, RBD-Fc, IFNα-RBD-Fc proteins by subcutaneous immunization, and the level of SARS-Cov2 S protein-specific antibody in serum was detected by ELISA method at specified time. , p<0.0001.

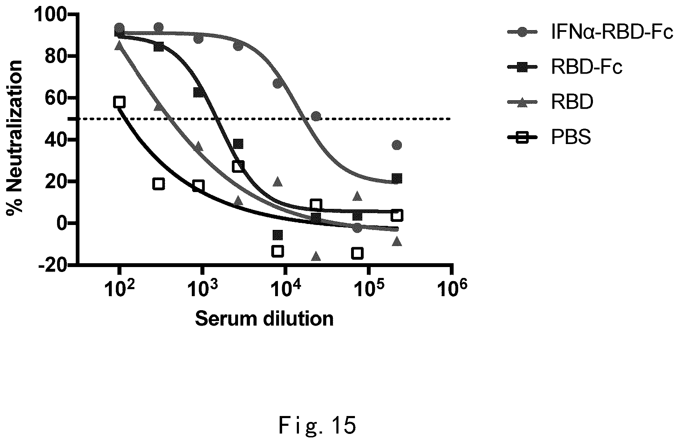

Figure 15 showed that the mice could produce high-titer antiviral serum after IFNα-RBD (SARS-CoV2)-Fc immunization, which could completely prevent SARS-CoV2 pseudovirus infection in *in vitro* cell experiments.

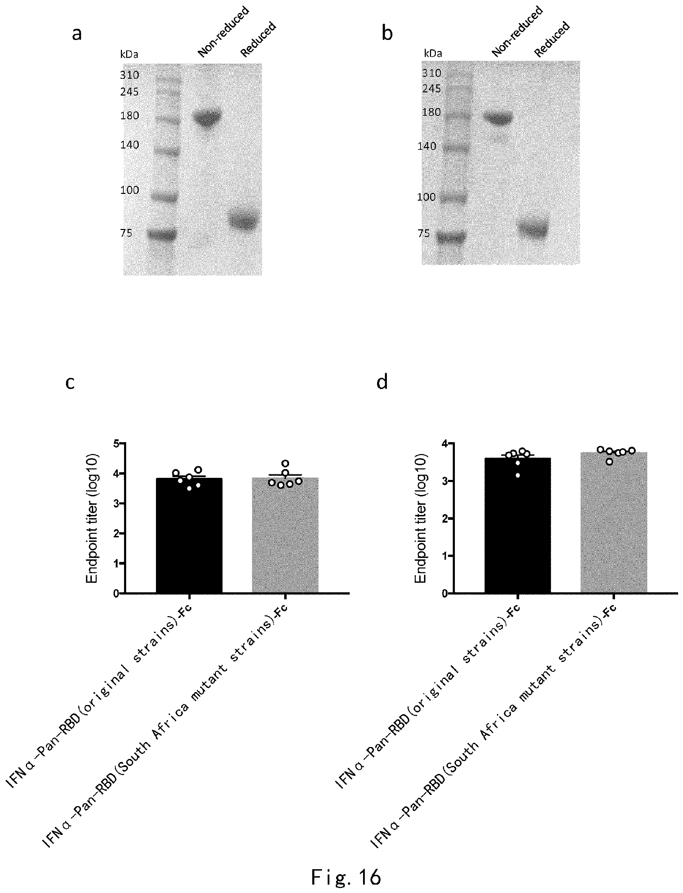

Figure 16 showed the detection of antiserum RBD-specific antibodies produced by IFNα-Pan-RBD (original strain)-Fc and IFNα-Pan-RBD (SARS-CoV-2 South Africa mutant strain)-Fc immunization. (a) SDS-PAGE electrophoresis map of IFNα-Pan-RBD (SARS-CoV-2 original strain)-Fc. (b) SDS-PAGE electrophoresis map of IFNα-Pan-RBD (SARS-CoV-2 South Africa mutant strain)-Fc. (c) Binding of RBD-specific antibody to the RBD of original strain 14 days after inoculation with IFNα-Pan-RBD (original strain)-Fc and IFNα-Pan-RBD (SARS-CoV-2 South Africa mutant strain)-Fc. (d) Binding of RBD-specific antibody to the RBD of South Africa mutant strain 14 days after inoculation with IFNα-Pan-RBD (original strain)-Fc and IFNα-Pan-RBD (SARS-CoV-2 South Africa mutant strain)-Fc.

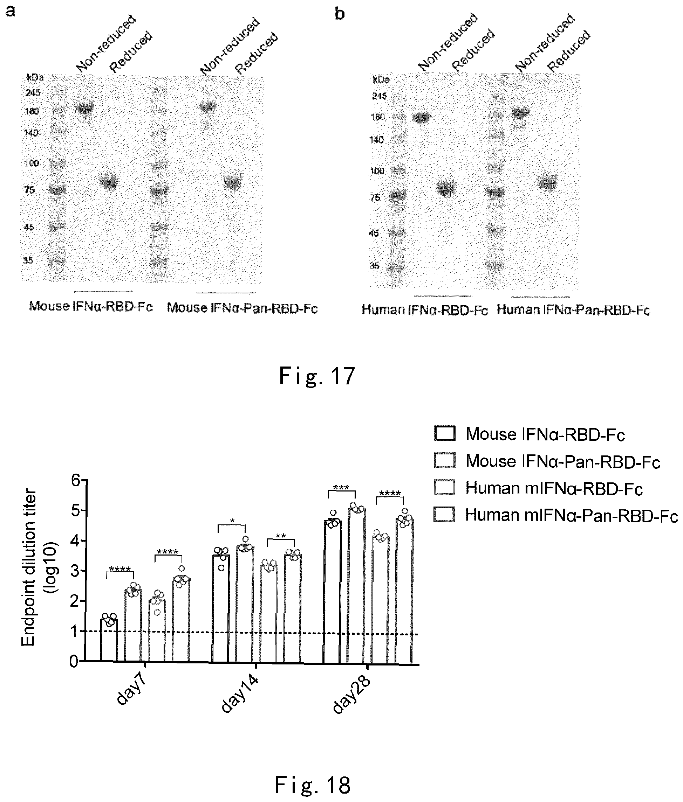

Figure 17 (a) SDS-PAGE electrophoresis maps of Mouse IFNα-RBD-Fc and Mouse IFNα-Pan-RBD-Fc proteins. (b) SDS-PAGE electrophoresis maps of Human IFNα-RBD-Fc, Human IFNα-Pan-RBD-Fc proteins.
Figure 18 showed that the Pan (Pan DR-binding epitope) CD4 T cell helper epitope could further enhance the immunogenicity of IFNα-RBD-Fc. The mice were inoculated with 10 µg Mouse IFNα-RBD-Fc,Mouse IFNα-Pan-RBD-Fc or 10 µg Human IFNα-RBD-Fc,Human IFNα-Pan-RBD-Fc proteins by intramuscular route, and a booster immunization was given at 14 days after inoculation. The mouse serum was respectively collected on the 7th, 14th, and 28th day after immunization, and the level of RBD-specific antibody in the mouse serum was detected by ELISA. * , p<0.05; ****, p<0.0001.

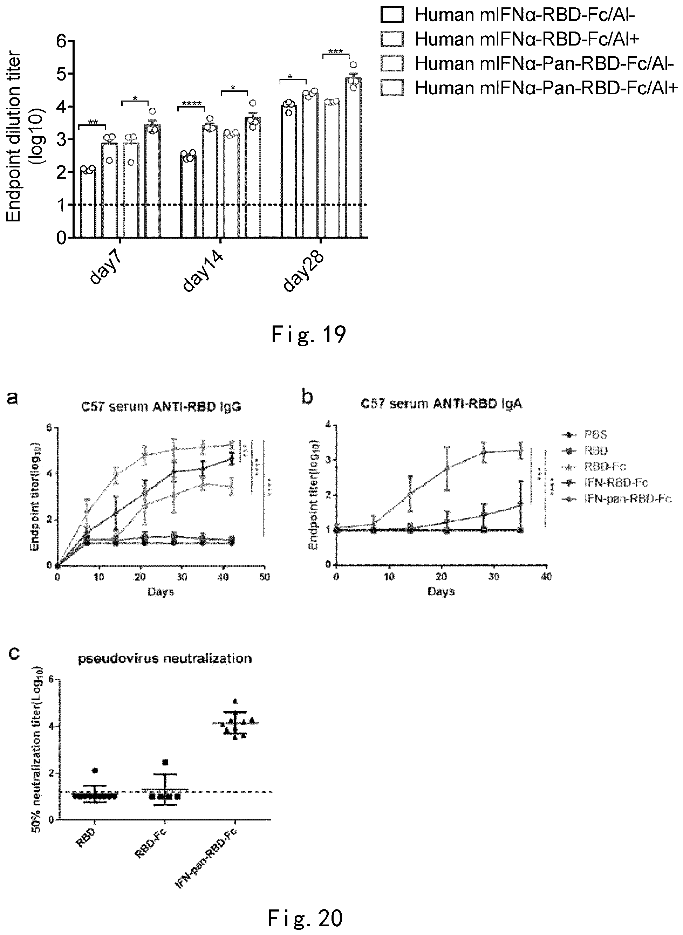

Figure 19 showed that the aluminum adjuvant could enhance specific humoral immune response induced by Human IFNα-RBD-Fc and Human IFNα-Pan-RBD-Fc proteins. C57BL/6 mice were inoculated with 10 µg Human IFNα-RBD-Fc or Human IFNα-Pan-RBD-Fc on day 0 and day 14 with (AL+) or without aluminum adjuvant (AL-); mouse serum was collected on the 7th, 14th, and 28th day after inoculation, and the level of SARS-CoV-2 RBD-specific antibody in the mouse serum was detected by ELISA. * , p<0.05; ****, p<0.0001.
Figure 20 showed that IFN-Pan-RBD-Fc intranasal inoculation could induce high titers of RBD-specific IgG and IgA neutralizing antibodies. C57BL/6 mice of 6-8 weeks old were divided into 5 groups, with 10 mice in each group, and were immunized with 10µg of IFNα-pan-RBD-Fc or the same molar amount of RBD, RBD-Fc, and IFNα-RBD-Fc proteins by intranasal immunization, and the intranasal dose was 10uL per mouse. Mice were immunized on day 0 and day 14 using two immunization procedures. The mouse serum was collected on the 7th, 14th, 21st, 28th, 35th, and 42nd days after immunization, and ELISA method was used to detect the levels of SARS-CoV-2 RBD-specific antibodies IgG(a) and IgA(b) in the serum of each group; the 42-day serum was collected for SARS-CoV-2 pseudovirus neutralization experiment in vitro (c). Statistical method: one-way ANOVA, *p<0.05 represented significant difference, **p<0.01, ***p<0.001, ****p<0.0001.

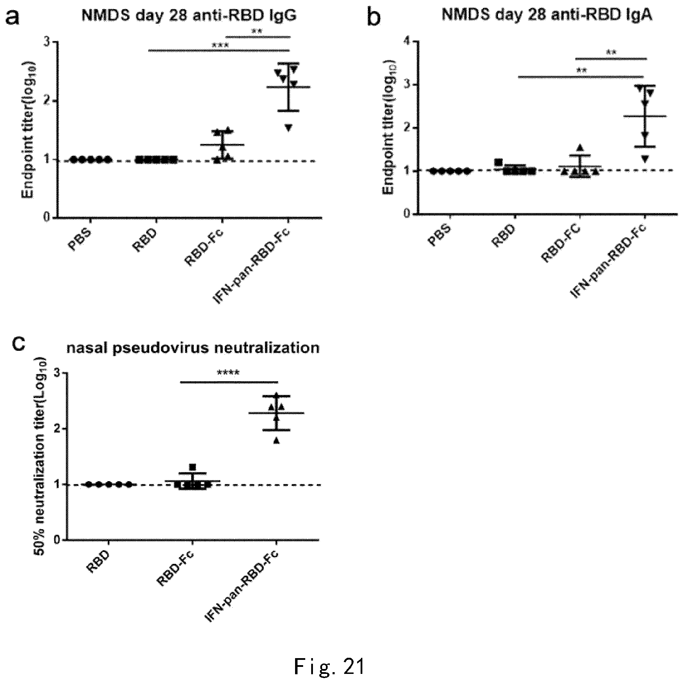

Figure 21 showed that intranasal inoculation of SARS-CoV-2 vaccine IFN-Pan-RBD-Fc without adjuvant induced high-titers RBD-specific IgG and IgA neutralizing antibodies in the nasopharynx and lung tissues. C57BL/6 mice of 6-8 weeks old were sacrificed at day 28 after immunization, and the nasal mucosa of the mice was taken and crushed with a tissue homogenizer. The homogenized liquid was centrifuged at 13,000 rpm for 10 minutes, and the supernatant was taken as the nasal mucosa supernatant (NMDS). ELISA method was used to detect the levels of SARS-CoV-2 RBD-specific antibodies IgG(a) and IgA(b) in the nasal mucosa supernatant (NMDS) of each group; the 28-day serum was collected for SARS-CoV-2 pseudovirus neutralization experiment in vitro (c). Statistical method: one-way ANOVA, *p<0.05 represented significant difference, **p<0.01, ***p<0.001, ****p<0.0001.

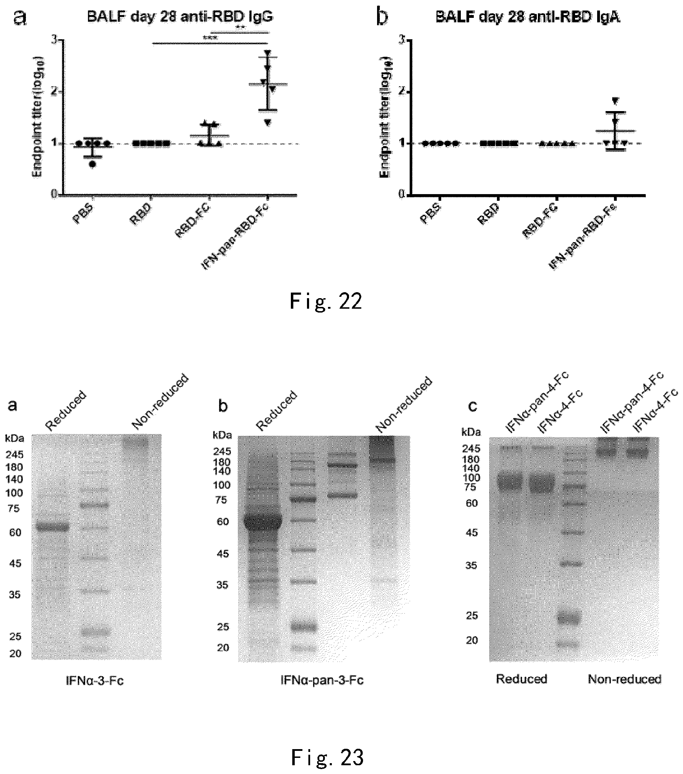

Figure 22 showed that intranasal inoculation of SARS-CoV-2 vaccine IFN-Pan-RBD-Fc without adjuvant induced high-titers RBD-specific IgG and IgA antibodies in lung tissues. 6-8 week old C57BL/6 mice were sacrificed at day 28 after immunization (as shown in Figure 19). For the lung of mice, a 1ml syringe was used to draw about 0.8ml of HBSS+100uMEDTA, injected into the endotracheal tube, blown and inhaled gently and repeatedly for three times, then the liquid was sucked out, collected into a centrifuge tube; the steps were repeated three times, and finally about 2ml of lung lavage fluid was obtained. Mouse lung lavage fluid was centrifuged at 500g for 5 minutes, and the obtained supernatant was the mouse lung lavage fluid (BALF). The ELISA method was used to detect the amounts of SARS-CoV-2 RBD-specific antibodies IgG (a) and IgA (b) in the lung lavage fluid (BALF) of mice in each group. Statistical method: one-way ANOVA, *p<0.05 represented significant difference, **p<0.01, ***p<0.001, ****p<0.0001.
Figure 23 showed the expression and purification of Her2 vaccine protein. Related proteins were expressed and purified in 293F cells, and the size and purity of the proteins were detected by SDS-PAGE and Coomassie brilliant blue staining. a. IFNα-3-Fc (62.6kDa); b. IFNα-pan-3-Fc (63.9kDa); c. IFNα-pan-4-Fc (74.9kDa) and IFNα-4-Fc (73.6kDa).

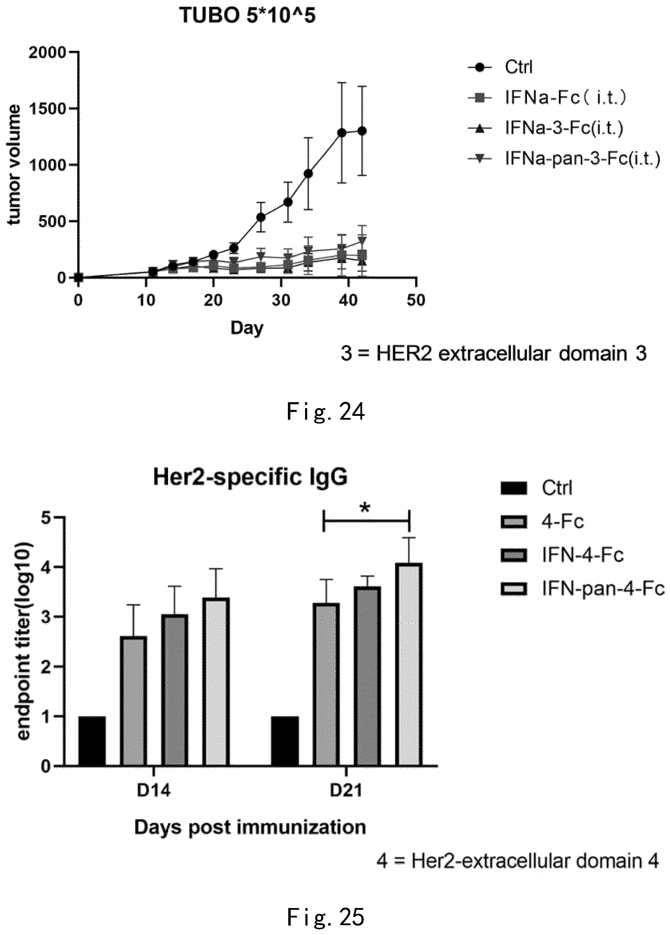

Figure 24 showed the analysis of antitumor activities of Her2 vaccines IFNα-3-Fc and IFNα-pan-3-Fc. TUBO breast cancer model mice were constructed, and were treated by intratumoral injection of related fusion proteins once a week for a total of 3 times when the tumor size was 50-80mm³. The dosage of IFNα-3-Fe was 10µg/dose/mouse, other fusion proteins were administered in equimolar amounts, and CpG was used as an adjuvant. The tumor size was measured, and the tumor growth curve was drawn.
Figure 25 showed that the function of IFNα and Pan enhanced the immunogenicity of Her2 antigen. BALB/C mice (n=5) of 6-8 weeks old were subcutaneously inoculated with mouse Her2 fusion protein vaccines 4-Fc, IFNα-4-Fc and IFNα-pan-4-Fc for immunization without adding adjuvant. The immunization dosage was 10µg/dose//mouse for IFNα-4-Fc, and other fusion proteins were inoculated in equimolar amounts. Venous blood was collected at day 14 and day 21 after immunization, and the antibody level of Her2-specific IgG in serum was detected by ELISA.

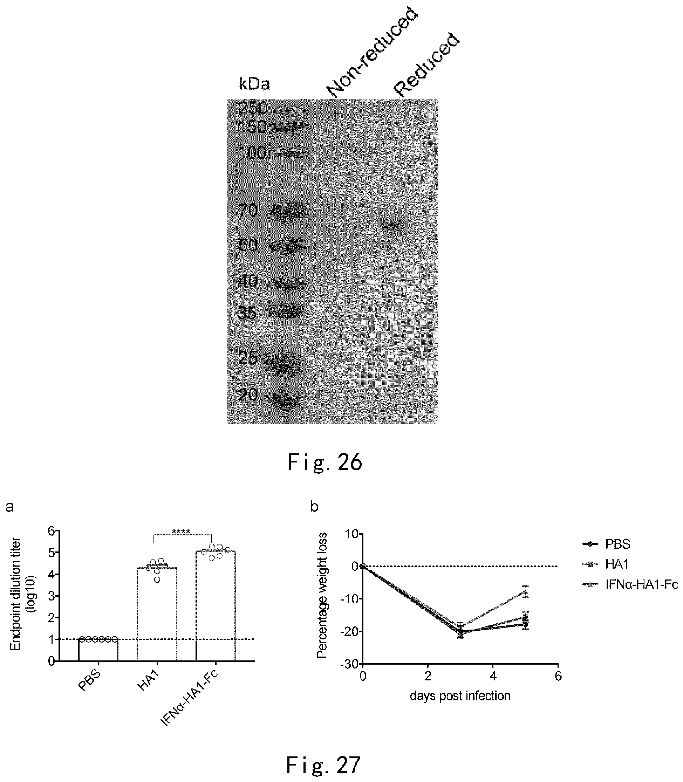

Figure 26 showed SDS-PAGE electrophoresis map of IFN-HA1-Fc fusion protein.
Figure 27 showed that mice were inoculated with 10µg of IFNα-HA1-Fc or the same molar amount of HA1 protein by intramuscular, and a booster immunization was performed 14 days after the initial inoculation. The mouse serum was collected on the 28th day after immunization, and the level of HA1-specific antibody in the mouse serum was detected by ELISA. The mice were infected with 1000 PFU of A/PR8 influenza virus by nasal infection on 42 days after immunization. From the third day after virus infection, the mice were observed and their body weight changes were recorded. (a) Mouse serum was collected 28 days after the initial immunization, and the level of HA1-specific antibody in mouse serum was detected by ELISA. (b) Body weight changes of mice after virus infection.
Figure 28:
   SDS-PAGE electrophoresis maps of IFNα-Pan-VZV-gE-Fc, IFNα-Pan-EBV-gp350-Fc, and IFNα-Pan-HSV-2-gD-Fc proteins.

### DETAILED DESCRIPTION OF THE INVENTION

In order to make the objective, technical solution and advantages of the present invention more clear, the present invention is described in detail below with reference to the examples and the accompanying drawings. The Examples are only illustrative of the present invention and are not intended to limit the scope of the present invention, and the Examples are only a part of the present invention, and do not represent all embodiments of the present invention. The scope of the invention is defined by the appended claims.

### Example 1. Design of vaccine platform

The vaccine platform of interferon-target antigen-immunoglobulin Fc (or antibody) consists of three structural units, wherein the first structural unit is interferon, the second structural unit is immunoglobulin Fc region (or antibody), and the third unit is target antigen. In the process of construction, the three structural units could be arbitrarily arranged and combined, and the target antigen could be connected to a Th cell helper epitope through a linker 2. The representative designs were as follows:

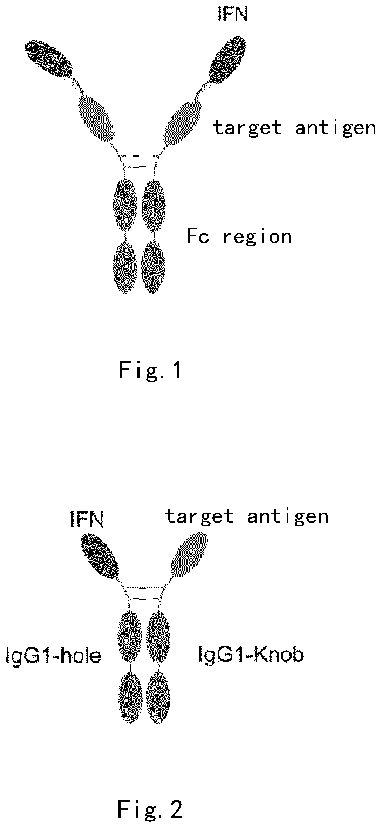

Figure 1 was a schematic diagram of the vaccine platform in the form of homodimer, arranged in the order of interferon-linking fragment 1-target antigen-immunoglobulin Fc.
Figure 2 was a schematic diagram of the vaccine platform in the form of heterodimer, according to the combination of interferon-linking fragment 1-IgG1-hole and target antigen-IgG1-knob, respectively.

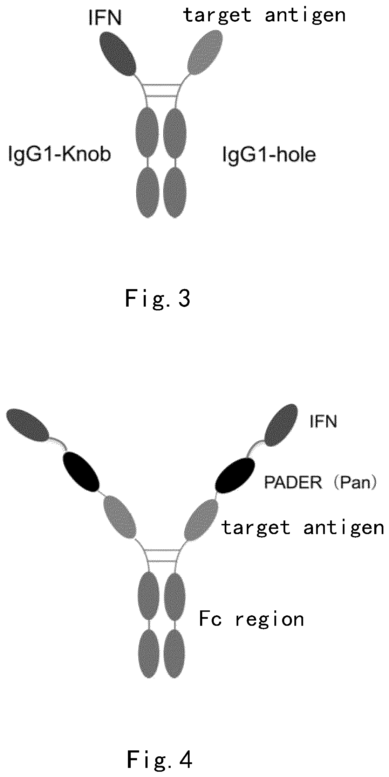

Figure 3 was a schematic diagram of the vaccine platform in the form of heterodimer, according to the combination of interferon-linking fragment 1-IgG1-knob and target antigen-IgG1-hole.

Next, the inventors tried to connect the target antigen to a cell helper epitope by a linking fragment 2, and then combine it with other two vaccine platform components. The representative designs were as follows:
Figure 4 was a schematic diagram of the vaccine platform in the form of homodimer, arranged in the order of interferon-linking fragment 1-Th cell helper epitope-linking fragment 2-target antigen-immunoglobulin Fc.

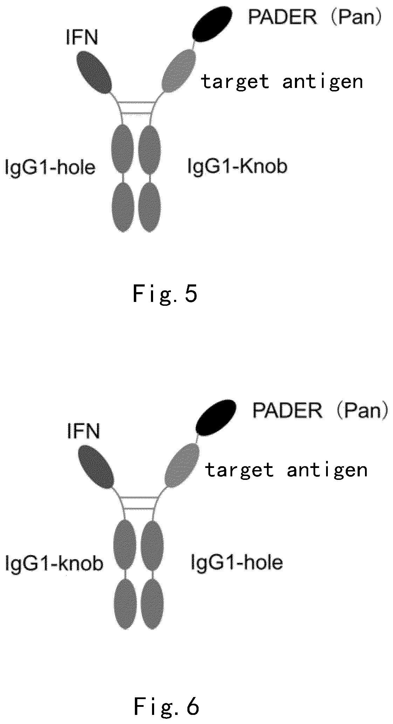

Figure 5 was a schematic diagram of the vaccine platform in the form of heterodimer, according to the combination of interferon-linking fragment 1-IgG1-hole and Th cell helper epitope-linking fragment 2-target antigen-IgG1-knob.
Figure 6 was a schematic diagram of the vaccine platform in the form of heterodimer, according to the combination of interferon-linking fragment 1-IgG1-knob and Th cell helper epitope-linking fragment 2-target antigen-IgG1-hole.

### Example 2. Construction, purification and production of the vaccine platform

The expression and production of the vaccine platform were described by taking hepatitis B virus Pres1 and coronavirus SARS-CoV-2 RBD protein homodimer as an example .

### 1. Vector construction, host cell transfection and induced expression

1.1. The vaccine structural units were constructed on PEE12.4 vector by molecular cloning to obtain a plasmid expressing the fusion protein, which was then transiently transfected into 293F cells, the culture supernatant was collected, and finally the protein of interest was purified by Protein A affinity chromatography.

Vector construction (taking HBV preS1 antigen as an example)
(1) PEE12.4-HindIII-signal peptide 1-interferon-BsiwI-Pres1-BstbI-hIgG1-EcoRI
(2) PEE12.4-HindIII-signal peptide 1-interferon-BsiwI-RBD(SARS-CoV-2)-BstbI-hIgG1-EcoRI
(3) PEE12.4-HindIII-signal peptide 1-interferon-Bsiwi-PADER-Pres1-hIgG1-EcoRI
(4) PEE12.4-HindIII-signal peptide 1-interferon-Bsiwi-PADER-RBD(SARS-CoV-2)-hIgG1-EcoRI

Linkers between each fragment of fusion protein were as follows:
(1) The linker between interferon and Pres1 was linking fragment 1
(2) The linker between interferon and RBD (SARS-CoV-2) was linking fragment 1
(3) The linker between interferon and PADER was linking fragment 1, and the linker between PADER and Pres1 was linking fragment 2
(4) The linker between interferon and PADER was linking fragment 1, and the linker between PADER and RBD (SARS-CoV-2) was linking fragment 2

1.2. Rapid expression of protein of interest by transient transfection:
(1) Cell thawing: Freestyle 293F cells were frozen in CD OptiCHOTM media (containing 10% DMSO) at a concentration of 10⁷ cells/ml. The cells were taken out from liquid nitrogen, and then dissolved quickly in a 37°C water bath, added into a 15ml centrifuge tube containing 10ml OptiCHOTM media, and centrifuged at 1,000rpm for 5min. The supernatant was discarded, and the cell pellet was suspended and cultured in 30ml OptiCHOTM media at 37°C, 8% CO₂, 135rpm. After 4 days, the cells were subjected to extended culture, and the concentration should not exceed 3 × 10⁶ cells/ml during the extended culture.
(2) Two days before transfection, the suspension cultured 293F cells were prepared for transient transfection (200ml) with an inoculum density of 0.6-0.8 × 10⁶ cells/ml.
(3) Two days later, the suspension of cells to be transfected was counted, and the estimated cell density was 2.5-3.5 × 10⁶ cells/ml, then the cell suspension was centrifuged at 1,000rpm for 5min, and the supernatant was discarded.
(4) Cells were resuspended with 50ml of fresh Freestyle 293 media, and centrifuged again at 1,000rpm for 5min, and the supernatant was discarded.
(5) 293F cells were resuspended with 200ml Freestyle 293 media.
(6) 600µg plasmids were diluted with 5ml of Freestyle 293 media, and filtered by a 0.22µM filter for sterilization.
(7) 1.8mg of PEI was diluted with 5ml of Freestyle 293 media and filtered with a 0.22µM filter for sterilization. Immediately thereafter, 5 ml of the plasmid and 5 ml of PEI were mixed, and allowed to stand at room temperature for 5 minutes.
(8) The plasmid/PEI mixture was added to the cell suspension, cultured in a 37°C, 8% CO₂, 85rpm incubator, and meanwhile supplemented with growth factor 50µg/L LONG^{™} R3IGF-1.
(9) After 4 hours, 200ml EX-CELLTM 293 media medium and 2mM Glutamine were supplemented, and then the cells were continued in culture at 135rpm.
(10) 24 hours later, 3.8mM of cell proliferation inhibitor VPA was added; 72 hours later, 40ml medium D was added, and then the cells were continued in culture; 6-8 days after transfection (the cell survival rate is less than 70%), the supernatant was collected for the next step of purification.

### 1.3. Collection, purification and electrophoresis verification of fusion protein

2. Purification of protein of interest by using Protein A:
(1) Sample preparation: the cell culture suspension was transferred to a 500ml centrifuge bucket, and centrifuged at 8,000rpm for 20min; precipitate was discarded; and supernatant was filtered by a 0.45µM filter to remove impurities, and then a final concentration of 0.05% NaN3 was added to prevent bacterial contamination during purification.
(2) Assembly of chromatographic column: An appropriate amount of Protein A Agarose (the amount was calculated by purifying 20 mg of human Fc fusion protein per 1 ml of Protein A) were mixed well, added to the chromatographic column, left at room temperature for about 10 minutes; after separation of Protein A and 20% ethanol solution, the outlet at the bottom was opened to allow the ethanol solution to flow out slowly by gravity.
(3) The chromatographic column was washed and equilibrated with 10 column volumes of distilled water and Binding buffer (20mM sodium phosphate + 0.15M NaCl, pH 7.0), respectively.
(4) The sample was loaded by a constant flow pump at a flow rate of 10 column volumes/hour, and flow-through was collected; and the sample was repeatedly loaded twice.
(5) The column was rinsed with more than 10 column volumes of Binding buffer to remove impurity proteins until no protein was detected in the effluent.
(6) The column was eluted by Elution Buffer (0.1 M Glycine, pH 2.7); eluent was collected in separate tubes, 1 tube for 1 ml eluent; and elution peaks were observed with a protein indicator solution (Bio-Rad protein assay). The collection tubes for the eluted peaks were mixed and added with an appropriate amount of 1 M Tris, pH 9.0 (to adjust the pH to 6-8, which should be more than 0.5 different from the isoelectric point of the purified protein).
(7) The protein of interest was substituted into required buffer by using Zeba desalting spin column or concentrating spin column (please be noted that the pH of the buffer should be adjusted to avoid the isoelectric point of the protein). BSA was used as a standard, and protein concentration was determined by SDS-PAGE electrophoresis and NanoDrop2000.
(8) After elution, the column was washed with 20 column volume of distilled water, and then with 10 column volume of 20% ethanol. Finally, the gel medium should be immersed in ethanol solution and stored at 4°C.

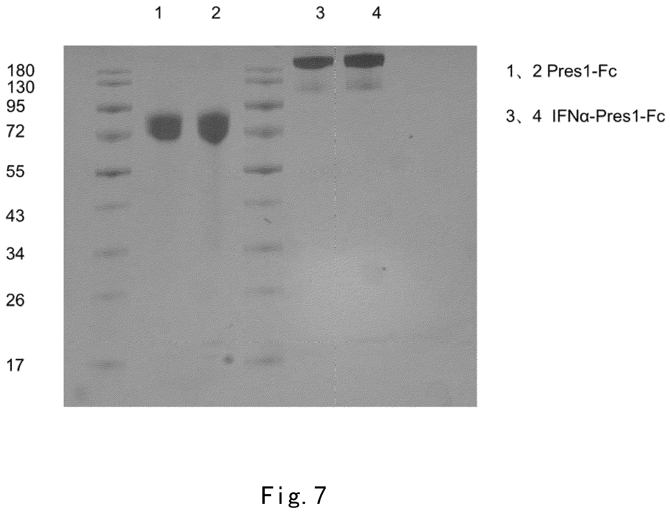

3. The SDS-PAGE electrophoresis map of the protein was shown in Figure 7.

### Example 3. IFNα-Pres1-Fc, Pres1-Fc could induce a stronger immune response in mice than Pres1 antigen alone.

Materials: C57BL/6 male mice (5-8 weeks old) were purchased from Beijing Vital River Laboratory Animal Technology Co., Ltd.; horseradish peroxidase (HRP)-labeled goat anti-mouse IgG was purchased from Beijing Kangwei Biology Technology Co., Ltd.; 96-well ELISA assay plate was purchased from Corning Costa; ELISA chromogenic solution was purchased from eBioscience; microplate reader SPECTRA max PLUS 384 was purchased from Molecular Company of the United States. The aluminum adjuvant was purchased from SIGMA.

Methods:
(1) The mice were immunized by Pres1 fusion protein; specially, 80 pmol IFN-Pres1-Fc or 80 pmol Pres1-Fc or Pres1 protein was mixed with aluminum adjuvant and subcutaneously administered to mice. At the designated time points, the serum of the mice was collected by taking blood from the orbit for antibody detection.
(2) The antibody produced by IFNα-Pres1-Fc had extensive neutralizing effect on different genotypes of HBV virus. 5-week-old male C57BL/6 mice were infected with 1x10¹¹ vg of AAV-HBV 1.3 (with HBV genotypes B, C, and D) through tail vein. After 6 weeks, mice with sustained and stable expression of HBV antigen were selected for the test. The selected mice (4 mice/group) were injected intravenously with serum from IFNα-Pres1-Fc immunized mice at 200ul/mouse. After 12 hours, the serum of the mice was collected, and the changes of the Pres1 antigen in the mice before and after the injection of the antiserum were detected by ELISA.
(3) Anti-Pres1 specific antibody in serum was detected by ELISA. Pres1 (2µg/ml) coating solution was added to the ELISA plate (Corning 9018) at 50ul per well, and the plate was coated at 4°C overnight. The plate was washed once with PBS, 260ul per well. The plate was blocked with 5% blocking solution (5% FBS) for two hours at 37°C. Serum samples were diluted with PBS (1: 10, 1: 100, 1:1000, 1:10000), added to the blocked ELISA plate at 50ul per well and incubated at 37°C for 1 hour. The plate was washed 5 times with PBST (260ul for each time), added with enzyme-labeled secondary antibody (enzyme-conjugated anti-mouse IgG-HRP 1:5000 diluted by PBS) at 50ul per well, and incubated at 37°C for 1 hour. The plate was washed 5 times with PBST (260ul for each time), added with substrate TMB 100ul/well, incubated at room temperature in the dark until color development; 50ul stop solution (2N H₂SO₄) was added to each well to stop color development, and the plate was read with a microplate reader, at OD450 -630.

Results: The immunogenicity of free Pres1 was weak, and the immunogenicity was greatly improved when the Pres1 was fused with IFNα and Fc moiety to form IFNα-Pres1-Fc fusion protein, which was shown in Figure 8(a). The antibody induced by IFNα-Pres1-Fc could produce a wide range of neutralizing effects on different HBV genotypes, as shown in Figure 8(b).

### Example 4. IFNα-Pres1-Fc could be used as a prophylactic vaccine against hepatitis B

Materials: C57BL/6 (6-8 weeks old) male mice were purchased from Beijing Vital River Laboratory Animal Technology Co., Ltd., and HBsAg detection kit was purchased from Shanghai Kehua Bio-Engineering Co., Ltd.. AAV-HBV 1.3 virus was purchased from Guangzhou PackGene Biotech Co., Ltd.. Other experimental materials were the same as those used in Example 3.

Methods:
(1) Mice were immunized subcutaneously with 80 pmol of different forms of Pres1 vaccines, including Pres1, Pes1-Fc, and IFNα-Pres1-Fc proteins. At day 28 after immunization, mice serum was collected and mice were infected with 1x10¹¹ vg AAV-HBV 1.3 virus, after that, mouse serum was collected every week for four weeks to detect anti-Pres1 antibody, HBsAg, and Pres1 antigen in the serum. At the third week, peripheral HBV-DNA levels of the mice were detected.
(2) ELISA detection of Pres1-specific antigen in serum. Antigen coating: Pres1 antibody XY007 (4µg/ml) coating solution was added to the ELISA plate (Corning 9018) at 50µl per well, and coated overnight at 4°C. The plated was washed once with PBS, 260µl per well. The plate was blocked with 5% blocking solution (5% FBS) for two hours at 37°C. Serum samples were diluted with PBS (1:10, 1:100), added to the blocked ELISA plate at 50µl per well (wherein, two duplicate wells were set for each dilution) and incubated at 37°C for 1 hour. The plate was washed 5 times with PBST (260µl for each time), added with 50µl enzyme conjugate (obtained from Kehua HBsAg Detection Kit) per well, and incubated at 37°C for 1 hour. The plate was washed 5 times with PBST (260µl for each time), added with substrate TMB 100µl/well, incubated at room temperature in the dark until color development; 50µl stop solution (2N H₂SO₄) was added to each well to stop color development, and the plate was read with a microplate reader, at OD450 -630.

Results: The mice in the IFNα-Pres1-Fc immunized group could produce a high level of Pres1 antibody before inoculation with the virus, and the antibody continued to maintain a high level during the virus infection, as shown in Figure 9 (a). Compared with the group without protein immunization, IFN-Pres1-Fc vaccine immunization could significantly prevent HBV infection, and the anti-preS 1 antibody produced after immunization could quickly and completely clear the preS1 antigen in the serum (Figure 9(b)), and most of the virus-infected mice in the IFN-Pres1-Fc immunized group were negative for peripheral HBsAg (Figure 9(c, d)). The above experimental results showed that IFN-Pres1-Fc as a vaccine could effectively prevent HBV infection, as shown in FIG. 9 .

### Example 5. IFNα-Pres1-Fc as a therapeutic vaccine for chronic B infection

Materials: C57BL/6 male mice (4 weeks old) were purchased from Beijing Vital River Laboratory Animal Technology Co., Ltd.. AAV-HBV 1.3 was purchased from Guangzhou PackGene Biotech Co., Ltd.. HBsAg detection kit was purchased from Shanghai Kehua Bio-Engineering Co., Ltd., and other experimental materials were the same as in those in Example 4.

Methods:
(1) Screening of HBV Carrier mice: 4-week-old HBV C57BL/6 mice were injected with 1x10¹¹ vg AAV-HBV 1.3 virus through tail vein, and HBV antigen HBsAg was detected in 1-6 weeks to screen mice with stable expression of HBsAg which were used as HBV carrier mice for experiments.
(2) The screened mice were subcutaneously injected with 80 pmol of different forms of Pres1 protein, once every two weeks for a total of three immunizations. The mouse serum was collected 14 days after immunization, and then collected once a week, and the levels of anti-Pres1 antibody, HBsAG, and Pres1 antigen in the mouse serum were detected by ELISA. HBV-DNA content in the peripheral blood of the mice was detected after the last blood collection.

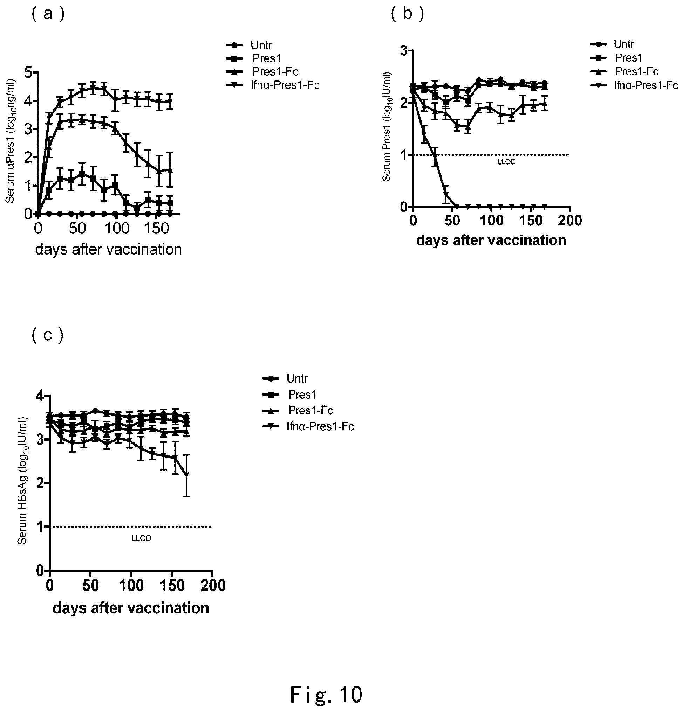

Results: We detected the preS1 antigen in the serum of Carrier mice immunized with IFN-Pres1-Fc vaccine, as well as the changes of Pres1 antibody and HBsAg in the serum. The results showed that after IFNα-Pres1-Fc vaccine immunization, high level of anti-Pres1 antibody in mice was produced, as shown in Figure 10(a), and the preS1 antigen in the serum could be completely eliminated, as shown in Figure 10(b). At the same time, HBsAg in the serum also decreased to a certain extent, as shown in Figure 10(c), while the untreated control group and the Pres1 vaccine immunization group alone had no therapeutic effects, as shown in Figure 10.

### Example 6. T cell helper epitopes enhanced the antibody response of IFNα-Pres1-Fc vaccine

### Materials: the same as those in Example 3

Methods:
(1) the mice were immunized by Pres1 fusion proteins, specially, 80 pmol IFN-Pan-Pres1-Fc containing Pan epitope or 80 pmol IFN-Pan-Pres1-Fc, Pres1-Fc, Pres1 protein were subcutaneously inoculated in mice. At the designated time points, the serum of the mice was collected by taking blood from the orbit for antibody detection.
(2) ELISA detection of anti-Pres1 specific antibody in serum, the same as that in Example 3.

Results: Compared with fusion protein vaccines such as IFN-preS1-Fc, the IFN-Pan-preS1-Fc could significantly enhance the immunogenicity of antigen molecules and induce the production of broad-spectrum neutralizing antibodies. C57/BL6 (n=8/group) mice were subcutaneously immunized with hepatitis B Pres1, Pres1-Fc, and IFNα-Pres1-Fc proteins without aluminum adjuvant, and the level of Pres 1-specific antibody in serum was detected by ELISA at specified time.

### Example 7. IFNa-Pan-Pres1-Fc as a therapeutic vaccine for chronic B infection

Materials: C57BL/6 male mice (4 weeks old) were purchased from Beijing Vital River Laboratory Animal Technology Co., Ltd.. AAV-HBV 1.3 was purchased from Guangzhou PackGene Biotech Co., Ltd.. HBsAg detection kit was purchased from Shanghai Kehua Bio-Engineering Co., Ltd., and other experimental materials were the same as in those in Example 4.

Methods:
(1) Screening of HBV Carrier mice: 4-week-old HBV C57BL/6 mice were injected with 1x10¹¹ vg AAV-HBV 1.3 virus through tail vein, and HBV antigen HBsAg was detected in 1-6 weeks to select mice with stable expression of HBsAg which were used as HBV carrier mice for experiments.
(2) The selected mice were subcutaneously injected with 80pmol of different forms of Pres1 protein, once every two weeks for a total of three immunizations. The mouse serum was collected 14 days after immunization, and then collected once a week, and the levels of anti-Pres1 antibody, HBsAg, and Pres1 antigen in the mouse serum were detected by ELISA. HBV-DNA content in the peripheral blood of the mice was detected after the last blood collection.

Results: We detected the preS1 antigen in the serum of Carrier mice immunized with IFNα-Pan-Pres1-Fc vaccine, as well as the changes of Pres1 antibody and HBsAg in the serum. The results showed that after IFN-Pan-Pres1-Fc vaccine immunization, the mice produced a high level of anti-Pres1 antibody, as shown in Figure 12(a). Moreover, the preS1 antigen in the serum could be completely eliminated, as shown in Figure 12(b), and the HBsAg in the serum also decreased to a certain extent, as shown in Figure 12(c), while the untreated control group and the Pres1 vaccine immunization group alone had no therapeutic effects. Moreover, the HBV DNA also decreased significantly in the IFNα-Pan-Pres1-Fc immunized group as shown in Figure 12(d).

### Example 8. The combination of IFNa-Pan-Pres1-Fc and HBsAg commercial vaccine broke immune tolerance against HBsAg and induced HBsAg-HBsAb serological conversion.

Materials: C57BL/6 male mice (4 weeks old) were purchased from Beijing Vital River Laboratory Animal Technology Co., Ltd.. AAV-HBV 1.3 was purchased from Guangzhou PackGene Biotech Co., Ltd.. HBsAg detection kit was purchased from Shanghai Kehua Bio-Engineering Co., Ltd., and Anti-HBsAg kit was purchased from Beijing Wantai Biological Pharmacy Co., Ltd. Commercial HBsAg vaccine was purchased from Amy Hansen Vaccine (Dalian) Co., Ltd. Other experimental materials were the same as those used in Example 7.

Methods:
(1) Screening of HBV Carrier mice: 4-week-old HBV C57BL/6 mice were injected with 1x10¹¹ vg AAV-HBV 1.3 virus through tail vein, and HBV antigen HBsAg was detected in 1-6 weeks to select mice with stable expression of HBsAg which were used as HBV carrier mice for experiments.
(2) The selected HBV Carrier mice were immunized with 80 pmol IFNα-pan-Pres1-Fc and 2 µg of commercial HBsAg vaccine at the same time for two consecutive times with an interval of 14 days between each time. The mouse serum was collected 14 days after the first immunization, and the mouse serum was collected every week thereafter, and the changes of anti-Pres 1, Pres1, anti-HBsAg, and HBsAg in the serum were detected. And when the mouse serum was collected for the last time, the level of HBV-DNA in the serum was detected.

RESULTS: We found that the combination of IFNα-Pan-Pres1-Fc with commercial HBsAg as a strategy for the treatment of chronic hepatitis B could eventually break HBsAg tolerance. The immune response generated in HBV-tolerant mice could completely clear the preS1 antigen in the serum, as shown in Figure 13(a), and there was a high concentration of Pres1 antibody in the serum (Figure 13(c)). Excitingly, the IFN-Pan-Pres1-Fc vaccine simultaneously effectively cleared the HBsAg in serum and induced partial serological conversion of HBsAb (Figures 13(b) and 4(d)), which were clinically considered as a key indicator of HBV cure. In addition, we detected the expression levels of HBV-related DNA in peripheral blood by fluorescence quantitative real-time PCR. The results showed that, compared with the control group, the immunization by the combination of IFNα-Pan-Pres 1-Fc and commercialized HBsAg could finally reduce the level of peripheral HBV DNA (Figure 13(e)). Based on the above results, we proposed a vaccine strategy for the treatment of chronic hepatitis B by the combination of IFNα-Pan-Pres1-Fc and commercial HBsAg vaccine.

### Example 9. IFNα-RBD(SARS-CoV2)-Fc could cause a stronger antibody response than free SARS-Cov2 RBD protein

Materials: Balb/c male and female mice (6-8 weeks) were purchased from Beijing Vital River Laboratory Animal Technology Co., Ltd., and the SARS-CoV-2 RBD protein was purchased from Beijing KEY-BIO Biotech Co.,Ltd. 293-hACE2 cells were provided by Professor Zhang Zheng (Shenzhen Third People's Hospital). Luciferase Reporter detection kit was purchased from Promega.

Other experimental materials were the same as those used in Example 3.

Methods:
(1) Mice were immunized with IFNα-RBD(SARS-Cov-2)-Fc fusion protein; specially, the mice were subcutaneously immunized with 10µg IFNα-RBD-Fc,RBD-Fc or 10µg RBD protein mixed with aluminum adjuvant. At 28 days after immunization, the serum of the mice was collected by taking blood from orbit for detection of SARS-Cov-2-specific antibodies.
(2) Detection of serum SARS-cov2 RBD antibody Antigen coating: RBD (1.5µg/ml) coating solution was added to the ELISA plate (Corning 9018) at 100µl per well, and coated overnight at 4°C. The plated was washed once with PBS, 260µl per well. The plate was blocked with 100µl of 5% blocking solution (5% FBS) for two hours at 37°C. Serum samples were diluted with PBS (1:10,1:100, 1:1000, 1:10000, 1:100000...), added to the blocked ELISA plate at 100µl per well and incubated at 37°C for 1 hour. The plate was washed 5 times with PBST (260µl for each time), added with enzyme-labeled secondary antibody (enzyme-conjugated anti-mouse IgG-HRP 1:5000 diluted by PBS) at 100µl per well, and incubated at 37°C for 1 hour. The plate was washed 5 times with PBST (260µl for each time), added with substrate TMB at 100µl/well, incubated at room temperature in dark for 15 minutes, waiting for the substrate to develop color. 50µl of stop solution (2N H₂SO₄) was added to each well to stop the color development, and the plate was read with a microplate reader, at OD450-630. Calculation of titer: the maximum dilution factor that was positive was selected, and the dilution factor was multiplied (X) by the OD value/Cutoff value (0.1) corresponding to the dilution factor, and the obtained value was the antibody titer corresponding to the serum.
(3) in vitro neutralization experiment of SARS-CoV-2 S protein pseudovirus. Antiserum was diluted by 1:3 and added to a 96-well plate, and 50µl pseudovirus particles with luciferase spike protein were added to the wells, the mixture of virus and antibody was left at 37°C for 1 hour, and 10^4 293-hACE2 cells per well were added to the 96-well plate. the 96-well plate was left in a 37°C cell culture incubator, and the activity of luciferase was detected after 48 hours.

Results: The immunogenicity of free SARS-CoV-2 RBD was weak, and the immunogenicity thereof was greatly improved when the IFNα and Fc were added to the SARS-CoV-2 RBD polypeptide protein region to form a IFNα-RBD-Fc fusion protein, as shown in Figure 14. Antibodies induced by IFNα-RBD-Fc could block the infection of cells by the pseudovirus of SARS-CoV-2 S protein in vitro, as shown in Figure 15.

### Example 10. Detection of antiserum RBD-specific antibodies produced by FNα-Pan-RBD (original strain)-Fc and IFNα-RBD (SARS-CoV-2 South Africa mutant strain)-Fc immunization.

Materials: Balb/c male and female mice (6-8 weeks) were purchased from Beijing Vital River Laboratory Animal Technology Co., Ltd., and the RBD protein of original SARS-CoV-2 strain was purchased from Beijing KEY-BIO Biotech Co.,Ltd. The RBD protein of the South Africa mutant strain of SARS-CoV-2 was purchased from Beijing Sino Biological Technology Co., Ltd.

Other experimental materials were the same as those used in Example 3.

Methods:
(1) the construction and expression of IFNα-Pan-RBD (original strain)-Fc and IFNα-RBD (SARS-CoV-2 South Africa mutant strain)-Fc protein were the same as those in Example 2.
(2) the mice were immunized with IFNα-Pan-RBD (original strain)-Fc and IFNα-Pan-RBD (SARS-CoV-2 South Africa mutant)-Fc fusion protein; specially, 10µg of IFNα-Pan-RBD (original strain )-Fc or IFNα-Pan-RBD (SARS-CoV-2 South Africa mutant strain)-Fc protein was mixed with aluminum adjuvant and subcutaneously inoculated in mice. At 14 days after immunization, the serum of the mice was collected by taking blood from orbit for detection of SARS-Cov-2-specific antibodies.
(3) Antibody response analysis by ELISA was the same as that in Example 9.

Results: The results of SDS-PAGE showed correct band size of IFNα-Pan-RBD (SARS-CoV-2 original strain)-Fc, indicating that the mutant SARS-CoV-2 IFNα-RBD (SARS-CoV-2 original strain)-Fc vaccine protein was successfully constructed, expressed and purified (Figure 16a). SDS-PAGE results showed correct band size of IFNα-Pan-RBD (SARS-CoV-2 South Africa mutant strain)-Fc, indicating successful construction, expression and purification of the mutant SARS-CoV-2 IFNα-RBD (SARS-CoV-2 South Africa mutant strain)-Fc vaccine protein (Figure 16b). ELISA results showed that the antibodies induced by immunization of mice with IFNα-Panan-RBD (original strain)-Fc and IFNα-Pan-RBD (SARS-CoV-2 South Africa mutant strain)-Fc could bind to the RBD protein of original SARS-CoV-2 strain, and there was no significant difference in the ability of binding to the RBD of the original strain (Figure 16c). Meanwhile, the ELISA results for South Africa mutant strain RBD also showed that the antibodies induced by immunization of mice with IFNα-Panan-RBD (original strain)-Fc and IFNα-Pan-RBD (SARS-CoV-2 South Africa mutant strain)-Fc could bind to the RBD of the South Africa mutant strain, and their binding abilities were equivalent (Fig. 16d).

### Example 11

Materials: C57BL/6 female mice (6-8 weeks old) were purchased from Beijing Vital River Laboratory Animal Technology Co., Ltd.. The SARS-CoV-2 RBD protein used in ELISA was purchased from Beijing KEY-BIO Biotech Co.,Ltd.; Mouse IFNα-RBD-Fc,Mouse IFNα-Pan-RBD-Fc, Human IFNα-RBD-Fc,and Human IFNα-Pan-RBD-Fc proteins were produced in-house and other experimental materials were the same as those in Example 3.

Methods:
(1) The fusion protein design, plasmid construction and protein purification methods were the same as those in Examples 1 and 2.
(2) Immunization of mice with vaccine proteins. 10 µg Mouse IFNα-RBD-Fc,Mouse IFNα-Pan-RBD-Fc or 10 µg Human IFNα-RBD-Fc,Human IFNα-Pan-RBD-Fc vaccine proteins were mixed with 20 µg aluminum adjuvant overnight, and then inoculated to mice through muscle immunization, and a booster immunization was carried out 14 days after the initial inoculation. The mouse serum was collected on the 7th, 14th, and 28th day after immunization, and the level of RBD-specific antibody in the mouse serum was detected by ELISA.
(3) Detection of serum SARS-cov2 RBD antibody. Antigen coating: RBD (1.5µg/ml) coating solution was added to the ELISA plate (Corning 9018) at 100µl per well, and coated overnight at 4°C. The plated was washed once with PBS, 260µl per well. The plate was blocked with 100µl of 5% blocking solution (5% FBS) for two hours at 37°C. Serum samples were diluted with PBS (1:10,1:100, 1:1000, 1:10000, 1:100000...), added to the blocked ELISA plate at 100µl per well and incubated at 37°C for 1 hour. The plate was washed 5 times with PBST (260µl for each time), added with enzyme-labeled secondary antibody (enzyme-conjugated anti-mouse IgG-HRP 1:5000 diluted by PBS) at 100µl per well, and incubated at 37°C for 1 hour. The plate was washed 5 times with PBST (260µl for each time), added with substrate TMB at 100µl/well, incubated at room temperature in dark for 15 minutes, waiting for the substrate to develop color. 50µl of stop solution (2N H₂SO₄) was added to each well to stop the color development, and the plate was read with a microplate reader, at OD450-630. Calculation of titer: the maximum dilution factor that was positive was selected, and the dilution factor was multiplied (X) by the OD value/Cutoff value (0.1) corresponding to the dilution factor, and the obtained value was the antibody titer corresponding to the serum.

### Result:

As shown in Figure 17, after the protein was expressed and purified, the SDS-PAGE results showed that the protein size was as expected and a single band was displayed at the target position.

As shown in Figure 18, the addition of Pan (Pan DR-binding epitope) CD4 T cell helper epitope could enhance the immunity of Mouse IFNα-RBD-Fc and Human IFNα-RBD-Fc. The experimental results showed that the addition of pan epitope could lead to higher production of RBD-specific antigen on day 7, day 14, or day 28 after vaccine protein immunization for Mouse IFNα-Pan-RBD-Fc compared with Mouse IFNα-RBD-Fc,and Human IFNα-Pan-RBD-Fc compared with Human IFNα-RBD-Fc.

### Example 12

Materials: C57BL/6 female mice (6-8 weeks old) were purchased from Beijing Vital River Laboratory Animal Technology Co., Ltd., and the SARS-CoV-2 RBD protein used in ELISA was purchased from Beijing KEY-BIO Biotech Co.,Ltd. The Human IFNα-RBD-Fc and Human IFNα-Pan-RBD-Fc proteins used for immunization were all produced in-house. Other experimental materials were the same as those used in Example 3.

Methods:
(1) Human IFNα-RBD-Fc and Human IFNα-Pan-RBD-Fc proteins were used to immunize mice. 10µg Human IFNα-RBD-Fc or Human IFNα-Pan-RBD-Fc protein was mixed with aluminum adjuvant overnight, as a vaccine sample containing aluminum adjuvant; for another group, 10µg Human IFNα-RBD-Fc or Human IFNα-Pan-RBD-Fc protein was diluted with PBS as a vaccine sample without adjuvant. In the presence or absence of aluminum adjuvant, mice were inoculated with 10 µg Human IFNα-RBD-Fc or Human IFNα-Pan-RBD-Fc proteins by intramuscular immunization, and then 14 days after inoculation a booster immunization was given. The mouse serum was collected on the 7th, 14th, and 28th day after immunization, and the level of RBD-specific antibody in the mouse serum was detected by ELISA.
(2) Detection of serum SARS-cov2 RBD antibody. Antigen coating: RBD (1.5µg/ml) coating solution was added to the ELISA plate (Corning 9018) at 100µl per well, and coated overnight at 4°C. The plated was washed once with PBS, 260µl per well. The plate was blocked with 100µl of 5% blocking solution (5% FBS) for two hours at 37°C. Serum samples were diluted with PBS (1:10, 1:100, 1:1000, 1:10000, 1:100000...), added to the blocked ELISA plate at 100µl per well and incubated at 37°C for 1 hour. The plate was washed 5 times with PBST (260µl for each time), added with enzyme-labeled secondary antibody (enzyme-conjugated anti-mouse IgG-HRP 1:5000 diluted by PBS) at 100µl per well, and incubated at 37°C for 1 hour. The plate was washed 5 times with PBST (260µl for each time), added with substrate TMB at 100µl/well, incubated at room temperature in dark for 15 minutes, waiting for the substrate to develop color. 50µl of stop solution (2N H₂SO₄) was added to each well to stop the color development, and the plate was read with a microplate reader, at OD450-630. Calculation of titer: the maximum dilution factor that was positive was selected, and the dilution factor was multiplied (X) by the OD value/Cutoff value (0.1) corresponding to the dilution factor, and the obtained value was the antibody titer corresponding to the serum.

### Result:

As shown in Figure 19, the application of aluminum adjuvant could enhance the immunogenicities of Human IFNα-RBD-Fc and Human IFNα-Pan-RBD-Fc proteins. Although the Human IFNα-RBD-Fc and Human IFNα-Pan-RBD-Fc vaccines without adjuvant could generate high-titer antibody responses, the Human IFNα-RBD-Fc and Human IFNα-Pan-RBD-Fc proteins with aluminum adjuvant could further improve the level of RBD-specific antibody response on the 7th day, 14th day, and 28th day after inoculation compared with the group without adjuvant.

### Example 13

### Materials:

The experimental animals C57BL/6 mice of 6-8 weeks old were purchased from Beijing Vital River Laboratory Animal Technology Co., Ltd., with the animal certificate number as No. 110011200106828974; the RBD protein for immunization was purchased from Beijing KEY-BIO Biotech Co.,Ltd.; RBD-Fc, IFNα-RBD-Fc and IFN-pan-RBD-Fc proteins were produced in-house; all adjuvants were purchased from SERVA, Germany; horseradish peroxidase (HRP) labeled goat anti-mouse IgG was purchased from Beijing Kangwei Biology Technology Co., Ltd.; 96-well ELISA assay plate was purchased from Corning Costar; ELISA chromogenic solution was purchased from eBioscience; the microplate reader SPECTRA max PLUS 384 was purchased from Molecular Company of the United States; tissue homogenizer was purchased from Beijing Heros Technology Co., Ltd.

### Methods:

Mice of 6-8 weeks old were divided into 5 groups, with 10 mice in each group, and were immunized with 10µg of IFNα-pan-RBD-Fc or the same molar amount of RBD, RBD-Fc, and IFNα-RBD-Fc proteins by intranasal immunization, and the intranasal dose was 10uL per mouse. Mice were immunized on day 0 and day 14 using two immunization procedures. The mouse serum was collected on the 7th, 14th, 21st, 28th, 35th, and 42nd days after immunization, and ELISA method was used to detect the content of SARS-CoV-2 RBD-specific antibodies in the serum of each group; the 28-day serum was collected for SARS-CoV-2 pseudovirus neutralization experiment in vitro.

### Result:

As shown in Figure 20, two nasal immunizations of RBD and RBD-Fc proteins could cause a certain degree of antibody response. The levels of serum IgG and IgA at the same time point induced by two nasal immunizations of IFNα-pan-RBD-Fc were significantly higher than those in RBD, RBD-Fc and IFN-RBD-Fc groups. The results of pseudovirus neutralization experiments showed that compared with RBD and RBD-Fc immunization groups, the IFN-RBD-Fc could induce a higher level of neutralizing antibodies.

### Example 14

### Materials:

The materials were the same as those in Example 10.

### Methods:

Mice of 6-8 weeks old were divided into 4 groups, with 5 mice in each group, and were immunized with 10µg of IFNα-pan-RBD-Fc or the same molar amount of RBD, RBD-Fc, and IFNα-RBD-Fc proteins by intranasal immunization, and the dose was 10uL per mouse. Mice were immunized on day 0 and day 14 using two immunization procedures. On the 28th day after immunization, the nasal mucosal supernatant and lung lavage fluid of the mice were collected, and the serum levels of SARS-CoV-2 RBD-specific antibodies in each group were detected by ELISA method, and the SARS-CoV-2 pseudovirus neutralization test was used to detect the neutralization experiment of SARS-CoV-2 pseudovirus in serum and nasal mucosal supernatant.

Obtaining of the supernatant of nasal mucosa and alveolar lavage fluid of mice used in immunization experiments: After the mice were killed in rest, the nasal mucosa of the mice was collected and crushed with a tissue homogenizer. The homogenized liquid was centrifuged at 13,000 rpm for 10 minutes, and the supernatant was taken as the nasal mucosa supernatant (NMDS). For the lung of mice, a 1ml syringe was used to draw about 0.8ml of HBSS+100uMEDTA, injected into the endotracheal tube, blown and inhaled gently and repeatedly for three times, then the liquid was sucked out, collected into a centrifuge tube; the steps were repeated three times, and finally about 2ml of lung lavage fluid was obtained. The mouse lung lavage fluid was centrifuged at 500g for 5 minutes, wherein the supernatant was the mouse lung lavage fluid (BALF), and the precipitate was the lymphocytes in the mouse lung, which could be further analyzed.

### Result:

As shown in Figure 21, compared with RBD and RBD-Fc protein, IFNα-pan-RBD-Fc protein could induce strong local IgG antibody response and IgA mucosal immunity in nasal mucosa after two nasal immunizations. The intensity of IFNα-pan-RBD-Fc protein response was stronger than that of RBD and RBD-Fc group. The results of the pseudovirus neutralization experiment showed that the IFNα-pan-RBD-Fcprotein immunization group could induce a higher titer of neutralizing antibodies in the nasal mucosa.

As shown in Figure 22, two nasal immunizations of C57BL/6 mice by IFNα-pan-RBD-Fc fusion protein also caused strong secretion of IgG antibody and IgA antibody in local lung tissue. The results of pseudovirus neutralization experiments showed that IFNα-pan-RBD-Fc induced a higher titer of neutralizing antibodies than RBD and RBD-Fc.

### Example 15

Her2 belongs to the HER family of type I transmembrane growth factor receptors and consists of an extracellular ligand-binding domain, a transmembrane domain and an intracellular tyrosine kinase domain. Once the ligand binds to the extracellular domain, the HER protein will dimerize and trans-phosphorylate its intracellular domain. The phosphorylated tyrosine residues can bind to a variety of intracellular signaling molecules, activate downstream signaling pathways, and regulate gene transcription. Most of the regulated genes are related to cell proliferation, survival, differentiation, angiogenesis, invasion and metastasis. The extracellular segment of Her2 protein is relatively large, with more than 600 amino acids, and can be divided into four domains, namely domains I, II, III, and IV. The currently approved Trastuzumab mainly binds to domain IV, Pertuzumab mainly binds to domain II, and the polypeptide vaccine E75, which is undergoing clinical trials, targets domain III. It shows that there are some important sites in different domains, which may mediate the anti-tumor effect. In order to study the vaccine platform for the prevention and treatment of tumors, the inventors selected the tumor antigen Her2 as a target, constructed IFN-Her2-Fc and IFN-Pan-Her2-Fc fusion protein vaccines, and then analyzed the anti-tumor activities and immunological activities of the vaccines in vivo.

### Materials and methods:

### Materials:

BALB/c female mice (6-8 weeks old) were purchased from Beijing Vital River Laboratory Animal Technology Co., Ltd.; TUBO cells were obtained from TCGA; other materials were the same as those in Example 3.

### Methods:

(1) The fusion protein design, plasmid construction and protein purification methods were the same as those in Examples 1 and 2.
   Firstly, expression plasmids were constructed for domains III and IV of the extracellular domain of mouse Her2 (respectively denoted as: IFNα-3-Fc, IFNα-pan-3-Fc, IFNα-pan-4-Fe and IFNα-4-Fc ), and then related proteins were expressed and purified in human 293F cell line. The protein size and purity were identified by SDS-PAGE and Coomassie brilliant blue staining.
(2) Analysis of direct antitumor activity of IFNα-3-Fc and IFNα-pan-3-Fc
   TUBO was a breast cancer cell line derived from BALB-NeuT mice, and was used to study the growth and treatment of Her2-positive breast cancers. Antitumor activity of IFNα in proteins was detected by using TUBO tumors. TUBO breast cancer model mice were constructed, 5*10⁵ TUBO cells were subcutaneously inoculated into BALB/C mice. The treatment was given once a week for a total of 3 times when the tumor size was 50-80mm³. The dosage of IFNα-3-Fe was 10µg/mouse, and other drugs were administered in equimolar amounts, and CpG was used as an adjuvant. The tumor size was measured, and the tumor growth curve was drawn.
(3) Analysis of the enhancement of immunogenicity of Her2 vaccine by IFNα and Pan
   BALB/C female mice aged 6-8 weeks were inoculated subcutaneously with HER2 domain V fusion protein vaccines 4-Fc, IFNα-4-Fc and IFNα-pan-4-Fc without adjuvant, once a week, 3 times in total. The immunization dosage was 10µg/mouse for IFNα-4-Fc, and other proteins were inoculated in equimolar amounts. Venous blood was collected at day 14 and day 21 after immunization, and the antibody level of Her2-specific IgG was detected by ELISA method.

Result:
(1) As shown in Figure 23, the size of the Her2 fusion protein was substantially as expected, and the purity was suitable for experimental requirements. IFNα-3-Fc (62.6kDa), IFNα-pan-3-Fc (63.9kDa), IFNα-pan-4-Fc (74.9kDa) and IFNα-4-Fc (73.6kDa). Under non-denaturing conditions, the proteins were in a dimer state, corresponding to the characteristics of automatic dimerization of Fc fragments.
(2) As shown in Figure 24, compared with the control group, intratumoral injection of Her2 fusion proteins IFNα-pan-3-Fc and IFNα-3-Fc significantly inhibited the growth of TUBO tumors, and the effect was comparable to that of the IFNα-Fc group. The above results showed that the activity of IFNα in the protein vaccine was good, and was not affected by factors such as steric hindrance, which can be used to further explore its efficacy and mechanism thereof in anti-tumor immunity.
(3) As shown in Figure 25, compared with the control group, 4-Fc, IFNα-4-Fc and IFNα-pan-4-Fc could all induce significant Her2 specific IgG antibody responses at day 14 and day 21 after immunization; wherein, the IFNα-4-Fc and IFNα-pan-4-Fc induced a trend toward increased antibody titers compared to the 4-Fc. Moreover, at day 21 after immunization, the antibody titer induced by IFNα-pan-4-Fc was significantly higher than that of the 4-Fc group. The above results showed that the addition of IFNα and pan could help increase the immunogenicity of 4-Fc and induce a stronger antigen-specific antibody response, and thus the IFN-Pan-HER2-Fc and IFN-Pan-HER2-Fc were potential effective anti-tumor vaccines against Her2 positive tumors.

### Example 16

Materials: BALB/c female mice (6-8 weeks old) were purchased from Beijing Vital River Laboratory Animal Technology Co., Ltd.; HA1 (A/PR8) protein used in ELISA was purchased from Beijing Sino Biological Technology Co., Ltd.; HA1 protein for immunization (A/PR8) was purchased from Beijing Sino Biological Technology Co., Ltd.; IFNα-HA1-Fc was produced in-house; H1N1 (A/PR8) influenza viruses used to infect mice were produced in-house; other experiments materials were the same as those in Example 3.

Methods:
(1) The IFNα-HA1-Fc protein design, plasmid construction and protein purification were the same as those in Examples 1 and 2.
(2) Immunization of mice by HA1 and IFNα-HA1-Fc proteins. 10µg IFNα-HA1-Fc or the same molar amount of HA1 protein was respectively mixed with 20 µg aluminum adjuvant overnight, and then inoculated to mice through muscle immunization, and a booster immunization was carried out 14 days after the initial inoculation. The mouse serum was collected on the 28th day after immunization, and the level of HA1-specific antibody in the mouse serum was detected by ELISA.
(3) Detection of serum HA1 antibodies. Antigen coating: HA1 (2µg/ml) coating solution was added to the ELISA plate (Corning 9018) at 100µl per well, and coated overnight at 4°C. The plated was washed once with PBS, 260µl per well. The plate was blocked with 100µl of 5% blocking solution (5% FBS) for two hours at 37°C. Serum samples were diluted with PBS (1:10, 1:100, 1:1000, 1:10000, 1:100000...), added to the blocked ELISA plate at 100µl per well and incubated at 37°C for 1 hour. The plate was washed 5 times with PBST (260µl for each time), added with enzyme-labeled secondary antibody (enzyme-conjugated anti-mouse IgG-HRP 1:5000 diluted by PBS) at 100µl per well, and incubated at 37°C for 1 hour. The plate was washed 5 times with PBST (260µl for each time), added with substrate TMB at 100µl/well, incubated at room temperature in dark for 15 minutes, waiting for the substrate to develop color. 50µl of stop solution (2N H₂SO₄) was added to each well to stop the color development, and the plate was read with a microplate reader, at OD450-630. Calculation of titer: the maximum dilution factor that was positive was selected, and the dilution factor was multiplied (X) by the OD value/Cutoff value (0.1) corresponding to the dilution factor, and the obtained value was the antibody titer corresponding to the serum.
(4) At day 42 after immunization, the mice were anesthetized and infected with 1000PFU A/PR8 influenza virus by nasal dripping. From the third day after infection, the mice were observed and weighed every two days.

### Result:

As shown in Figure 26, after protein expression and purification, the result of protein size and purity by SDS-PAGE showed a single band at the position of the band size of interest. As shown in Figure 27, IFNα-HA1-Fc could induce a higher titer of HA1-specific antibodies than HA1 protein (Figure 27a), indicating that the vaccine platform could enhance the immunogenicity of HA1 protein. After the mice were challenged, there would be a significant change in the body weight thereof. However, compared with the PBS group and the HA1 protein immunization group, the body weight of the mice in the IFNα-HA1-Fc immunization group would recover rapidly, indicating that IFNα-HA1-Fc vaccine immunization showed good protection against influenza infection (Figure 27b).

### Example 17

### Materials and methods:

The designs, plasmid constructions and protein purifications of IFNα-Pan-VZV-gE-Fc, IFNα-Pan-EBV-gp350-Fc, and IFNα-Pan-HSV-2-gD-Fc proteins were the same as those in Examples 1 and 2.

### Result:

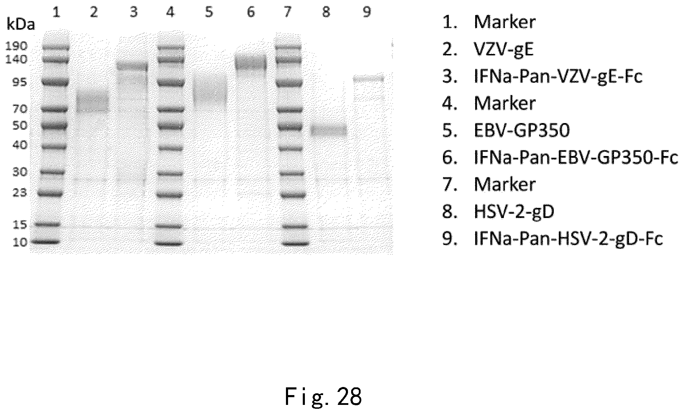

As shown in Figure 28, after expression and purification of IFNα-Pan-VZV-gE-Fc, IFNα-Pan-EBV-gp350-Fc, and IFNα-Pan-HSV-2-gD-Fc fusion proteins, the results of protein size and purity by SDS-PAGE showed that the band of interest were at correct positions.
Additional aspects and embodiments are set out in the following numbered paragraphs which form part of the description.
1. A vaccine, which comprises a fusion protein containing an interferon-target antigen-immunoglobulin Fc region (or antibody Ab) as structural unit,
   wherein the interferon is the first structural unit, which can be type I interferon, type II interferon and/or type III interferon such as IFN-α, IFN-β, IFN-γ, IFN-λ1 (IL-29) , IFN-λ2 (IL-28a), IFN-λ (IL-28b) and IFN-ω; the interferon can be derived from human or mouse; preferably the interferon is type I interferon, such as IFN-α , such as mouse IFN-α4, human IFN-α2, mutants of human IFN-α2 (binding to human and mouse IFN receptors), for example, as shown in SEQ ID NO.1, SEQ ID NO.21, SEQ ID NO.22,
   wherein the target antigen is the third structural unit; the target antigen may be, for example, a tumor antigen, a pathogen antigen, such as a viral or bacterial antigen; wherein the target antigen may be, for example, a mutated target antigen different from the wild type, including for example, natural point mutations/deletion mutations/addition mutations/truncations, artificial point mutations/deletion mutations/addition mutations/truncations of wild-type antigens, any combination of natural or artificial mutations, and subtypes produced after mutations, wherein the virus can be, for example, SARS-COV-2, or wherein the target antigen can be, for example, full length or S1 region of the SARS-COV-2 virus S protein, for example, the target antigen can be antigens as shown in SEQ ID NO.76 or SEQ ID NO.77,
   wherein the immunoglobulin Fc region (or antibody) is the second structural unit, which may be the constant region of IgG1, IgG2, IgG3, IgG4 and/or IgM, such as Fc region of IgG1, and Fc region as shown in SEQ ID NO.2, SEQ ID NO.23, and SEQ ID NO.24 of IgG1-Fc-hole and IgG1-Fc-knob used to form a heterodimer; wherein the antibody as the second structural unit (including, for example, antibody heavy and light chains, or single-chain antibodies, referred to as Ab) may be antibodies for DC targeting activation, including anti-PD-L1, anti-DEC205, anti-CD80/86 and other antibodies,
   Optionally, the vaccine may be a targeting vaccine, and optionally, the fusion protein may also contain one or more Th cell helper epitopes and/or linking fragments.
2. The vaccine of paragraph 1, wherein the target antigen is a virus antigen, and the virus may be for example HBV, HPV, VZV, EBV, HSV-2, HIV, influenza virus, coronavirus, such as SARS-COV, SARS-COV-2, MERS-CoV, for example, said antigen may be HBV antigen, such
   as HBV Pres1 antigen, HBsAg antigen or peptide fragments, such as ad subtype or ay subtype HBV Pres1 antigen, such as ad subtype HBV Pres1 antigen as shown in SEQ ID NO.6, such as ay subtype HBV Pres1 antigen as shown in SEQ ID NO.26; for example, HBV HBsAg antigen (including various subtypes and peptide fragments), such as adr subtype HBV HBsAg antigen as shown in SEQ ID NO.7, such as adw subtype HBV HBsAg antigen as shown in SEQ ID NO.27, such as ayw subtype HBV HBsAg antigen as shown in SEQ ID NO.28; for example, the antigen can be, for example, a SARS-COV-2 antigen, such as a SARS-COV2 RBD antigen, such as the SARS-COV2 RBD antigen as shown in SEQ ID NO.8; for example an influenza virus antigen, such as an influenza virus HA antigen, such as influenza virus HA antigen as shown in SEQ ID NO.9; for example HPV antigen, such as HPV E7 antigen as shown in SEQ ID NO. 10; for example gE antigen, such as herpes zoster virus (VZV) gE antigen as shown in SEQ ID NO.91; for example EBV-gp350, such as Epstein-Barr virus (EBV) gp350 protein as shown in SEQ ID NO.92; for example gD antigen, such as herpes simplex virus 2 (HSV-2) gD antigen as shown in SEQ ID NO.93; said antigen may be, for example, EBV EBNA1/LMP2, VZV-IE62, HSV-2 ICPO, HIV gp120 antigen;
   wherein the target antigen may be a mutated virus antigen, such as a mutant of any virus antigens, such as a mutant of SARS-COV-2, including for example natural point mutations/deletion mutations/addition mutations/truncations, artificial point mutations/deletion mutations/addition mutations/truncations, any combination of natural or artificial mutations, subtypes generated by mutations, derived from SARS-COV-2 protein (such as one or more of S protein, N protein, M protein, E protein); for example, the mutated virus antigen can be mutants of full length of S protein (SEQ ID NO.76), S1 region (SEQ ID NO.77), RBD region (SEQ ID NO. 78) of wild-type SARS-COV-2; for example, the mutated virus antigen may comprise one or more of the following mutations of S protein of SARS-COV-2: NTD region 69-70 deletion, Y144 deletion, 242-244 deletion, L18F, D80A, D215, R246I mutations, RBD region K417, E484, N501Y, L452R mutations, D614G, H655Y mutations; for example, the mutated virus antigen may comprises mutations present in British B.1.1.7 (501Y.1) mutant strain, South Africa B.1.351 (501Y.2) mutant strain and Brazil P1 (501Y.3) mutant strain, California B.1.429 mutant strain; for example, the mutated virus antigen may comprise a mutant shown in any of SEQ ID NO.79, SEQ ID NO .80, SEQ ID NO.81, SEQ ID NO.82; for example, the mutated virus antigen may comprise a mutant shown in any of SEQ ID NO.79, SEQ ID NO.80, SEQ ID NO. 81, SEQ ID NO.82,
   the virus antigen can be fused to an helper polypeptide epitope that is expressed to enhance the response of B cells and T cells, and can be located at the N-terminal or C-terminal of the antigenic epitope, such as Pan HLA DR-binding epitope (PADER), such as the amino acid sequence as shown in SEQ ID NO. 3;
   the linking fragments of each structural unit are flexible polypeptide sequences, and can be linking fragments 1 and 2, for example as shown in the amino acid sequences of SEQ ID NO.4 and SEQ ID NO.25,
   the N-terminal of each polypeptide sequence composed of the structural units may comprise a corresponding signal peptide capable of promoting protein secretion, for example as shown in the amino acid sequence of SEQ ID NO.5,
   the vaccine can be produced by eukaryotic expression systems, for example, by eukaryotic expression system 293F, and CHO cells.
3. The vaccine of paragraph 1 or 2, wherein the target antigen is a tumor antigen, such as a protein molecule highly expressed by tumor cells, for example, the antigen can be human epidermal growth factor receptor 2 (HER2/neu) and epidermal growth factor receptor (EGFR);
   for example, protein molecule Her2 highly expressed by tumor cells and various functional regions and truncations thereof, such as antigens or mutants thereof as shown in SEQ ID NO. 85, 86, 97, 88, 89, 90.
4. The vaccine of any one of paragraphs 1-3, wherein the fusion protein is a homodimer or heterodimer fusion protein, optionally the fusion protein can also comprises one or more Th cell helper epitopes and/or linking fragments in any one or two chains (i.e. a first polypeptide chain and/or or a second polypeptide chain) of the homodimer or heterodimer,
   optionally, the homodimer fusion protein comprises a first polypeptide chain and a second polypeptide chain, wherein the first polypeptide chain and the second polypeptide chain are identical, for example, the first polypeptide chain and the second polypeptide chain each comprises an IFN, a target antigen, and an immunoglobulin Fc region (or Ab) in sequence from N-terminal to C-terminal, or the three structural units are combined in any order to generates a homodimer; preferably, the first polypeptide chain and the second polypeptide chain each comprises an IFN, a target antigen, and an immunoglobulin Fc region (or Ab) in sequence from N-terminal to C-terminal; the fusion protein may also comprise a Th cell helper epitope; optionally the heterodimer fusion protein comprises a first polypeptide chain and a second polypeptide chain, wherein the first polypeptide chain and the second polypeptide chain are different, for example the first polypeptide chain may comprise an IFN and an immunoglobulin Fc region (or Ab) in sequence from N-terminal to C-terminal, or comprise an immunoglobulin Fc region (or Ab) and an IFN in sequence from N-terminal to C-terminal; the second polypeptide chain may comprise a target antigen and an immunoglobulin Fc region (or Ab), wherein the target antigen may be located at the N-terminal, and the immunoglobulin Fc region (or Ab) may be located at the C-terminal, or the immunoglobulin Fc region (or Ab) may be located at the N-terminal, and the target antigen may be located at the C-terminal; or the three structural units are combined in any order to generates a heterodimer; preferably, the IFN and the target antigen are respectively located at the N-terminal of the two polypeptides, and the immunoglobulin Fc region (or Ab) is located at the C-terminal of the two polypeptides; the fusion protein may also comprise a Th cell helper epitope.
5. The vaccine of paragraph 4, wherein
   1) The first polypeptide and the second polypeptide of the homodimer may comprise the amino acid sequences as shown in SEQ ID NO.11, 12, 13, 14, 29, 30, 31, 32, 38, 39, 40, 47, 48 , 49, 50, 51, 56, 57, 59, 58, 65, 66, 67, 68,
   2) The first polypeptide of the heterodimer may comprise the amino acid sequences as shown in SEQ ID NO.15, 33, 42, 51, 60, and 69, and the second polypeptide may comprise the amino acid sequences as shown in SEQ ID NO. 16, 17, 18, 19, 34, 35, 36, 37, 43, 44, 45, 46, 52, 53, 54, 55, 61, 62, 63, 64, 70, 71, 72, 73,
   3) The antibody may comprise DC targeting antibodies, immune checkpoint blocking antibodies, immune activation antibodies, etc., for example, vaccines containing anti-PD-L1 antibody (SEQ ID NO.20), anti-DEC205 antibody, anti-CD80/86 antibody, etc..
6. A nucleic acid molecule encoding the fusion protein in the vaccine of any one of paragraphs 1-5, an expression vector comprising the nucleic acid molecule, or a host cell, such as an eukaryotic cell, comprising the nucleic acid molecule or the expression vector.
7. Use of the fusion protein in the vaccine of any one of paragraphs 1-5 in the preparation of a composition or kit, such as a pharmaceutical or immunogenic composition or kit, a recombinant microorganism or cell line.
8. The use of paragraph 7, wherein the composition or kit is used for the prevention or treatment of tumors or pathogens, such as the prevention or treatment of viruses or bacteria, wherein the viruses can be HBV, HPV, EBV, influenza virus , HIV, coronaviruses, such as SARS-COV, SARS-COV-2, MERS-CoV; for example, the composition or kit is used as a prophylactic or therapeutic vaccine for hepatitis B, HBV, influenza, SARS-COV2, HPV, HPV-related tumors, EBV, EBV-related tumors, or HIV.
9. The vaccine of any one of paragraphs 1-5 or the use of paragraph 7 or 8, wherein the vaccine, the composition or the kit can be inoculated by intramuscular, intravenous, transdermal, subcutaneous or nasal or other immunization routes, wherein the vaccine, the composition or the kit can also comprise an adjuvant, and the adjuvant can comprise aluminum adjuvant (Alum), Toll-like receptor 4 activator ligand MPLA, Toll-like receptor 9 ligand, oligodeoxynucleotide (CpG-ODN), M59 and Freund's adjuvant.
10. The vaccine of any one of paragraphs 1-5 or the use of paragraph 7 or 8, wherein the vaccine can be used in combination with other prophylactic or therapeutic therapies; for example, the vaccine can be HBV therapeutic vaccine, which can be used in combination with another prophylactic or therapeutic HBV therapy, for example, the HBV therapeutic vaccine can be used in combination with hepatitis B virus envelope protein HBsAg vaccine, for example for the treatment of chronic hepatitis B virus infection, for example, the HBV therapeutic vaccine can be combined with nucleoside or nucleotide analogues, for example for the treatment of chronic hepatitis B virus infection, for example the prophylactic or therapeutic vaccines for influenza, SARS-COV2, HPV, EBV, HIV can be used in combination with antiviral drugs and other treatment methods; the prophylactic or therapeutic vaccines for HPV, EBV-related tumors can be used in combination with antiviral and antitumor drugs and therapies; for example, the vaccine of any one of paragraphs 1-5 can be combined with other vaccines for viruses or pathogens or tumors to form a multivalent vaccine, for example the SARS-COV-2 vaccine of any one of paragraphs 1-5 can be combined with other vaccines such as influenza vaccine to form a multivalent vaccine; for example, the vaccine of any one of paragraphs 1-5 and an adenovirus vaccine or mRNA vaccine or inactivated vaccine or DNA vaccine for the same virus, pathogen, or tumor are inoculated in sequence or at the same time, for example, the SARS-COV-2 fusion protein vaccine and adenovirus vaccine or mRNA vaccine or inactivated vaccine or DNA vaccine for SARS-COV-2 are inoculated in sequence or at the same time, for example, the sequence of inoculation can be as follows: 1) firstly, the SARS-COV-2 fusion protein vaccine of the present invention, and secondly the adenovirus vaccine or mRNA vaccine or inactivated vaccine or DNA vaccine for SARS-COV-2; 2) firstly, the adenovirus vaccine or mRNA vaccine or inactivated vaccine or DNA vaccine for SARS-COV-2, and secondly the SARS-COV-2 fusion protein vaccine; 3) the SARS-COV-2 fusion protein vaccine and the adenovirus vaccine or mRNA vaccine or inactivated vaccine or DNA vaccine for SARS-COV-2 are inoculated at the same time.

### References

[1] WHO W. Global hepatitis report 2017 [J]. Geneva: World Health Organization, 2017,
[2] RAZAVI-SHEARER D, GAMKRELIDZE I, NGUYEN M H, et al. Global prevalence, treatment, and prevention of hepatitis B virus infection in 2016: a modelling study [J]. The lancet Gastroenterology & hepatology, 2018, 3(6): 383-403.
[3] SCHWEITZER A, HORN J, MIKOLAJCZYK R T, et al. Estimations of worldwide prevalence of chronic hepatitis B virus infection: a systematic review of data published between 1965 and 2013 [J]. The Lancet, 2015, 386(10003): 1546-55.
[4] VOS T, ABAJOBIR A A, ABATE K H, et al. Global, regional, and national incidence, prevalence, and years lived with disability for 328 diseases and injuries for 195 countries, 1990-2016: a systematic analysis for the Global Burden of Disease Study 2016 [J]. The Lancet, 2017, 390(10100): 1211-59.
[5] KRAMMER F, SMITH G J D, FOUCHIER R A M, et al. Influenza [J]. Nature reviews Disease primers, 2018, 4(1): 3.
[6] HANNOUN C. The evolving history of influenza viruses and influenza vaccines [J]. Expert review of vaccines, 2013, 12(9): 1085-94.
[7] HOUSER K, SUBBARAO K. Influenza vaccines: challenges and solutions [J]. Cell host & microbe, 2015, 17(3): 295-300.
[8] HU B, GUO H, ZHOU P, et al. Characteristics of SARS-CoV-2 and COVID-19 [J]. Nature Reviews Microbiology, 2020, 1-14.
[9] LURIE N, SAVILLE M, HATCHETT R, et al. Developing Covid-19 vaccines at pandemic speed [J]. New England Journal of Medicine, 2020, 382(21): 1969-73.
[10] LI Q, WU J, NIE J, et al. The impact of mutations in SARS-CoV-2 spike on viral infectivity and antigenicity [J]. Cell, 2020, 182(5): 1284-94. e9.
[11] KONTERMANN R E. Strategies for extended serum half-life of protein therapeutics [J]. Current opinion in biotechnology, 2011, 22(6): 868-76.
[12] MEKHAIEL D N, CZAJKOWSKY D M, ANDERSEN J T, et al. Polymeric human Fc-fusion proteins with modified effector functions [J]. Scientific reports, 2011, 1(1): 1-11.
[13] ROOPENIAN D C, AKILESH S. FcRn: the neonatal Fc receptor comes of age [J]. Nature reviews immunology, 2007, 7(9): 715-25.
[14] VILCEK J. Fifty years of interferon research: aiming at a moving target [J]. Immunity, 2006, 25(3): 343-8.
[15] BRACCI L, LA SORSA V, BELARDELLI F, et al. Type I interferons as vaccine adjuvants against infectious diseases and cancer [J]. Expert review of vaccines, 2008, 7(3): 373-81.
[16] HAHM B, TRIFILO M J, ZUNIGA E I, et al. Viruses evade the immune system through type I interferon-mediated STAT2-dependent, but STAT1-independent, signaling [J]. Immunity, 2005, 22(2): 247-57.
[17] ITO T, AMAKAWA R, INABA M, et al. Differential regulation of human blood dendritic cell subsets by IFNs [J]. The Journal of Immunology, 2001, 166(5): 2961-9.
[18] MONTOYA M, SCHIAVONI G, MATTEI F, et al. Type I interferons produced by dendritic cells promote their phenotypic and functional activation [J]. Blood, 2002, 99(9): 3263-71.
[19] LE BON A, ETCHART N, ROSSMANN C, et al. Cross-priming of CD8+ T cells stimulated by virus-induced type I interferon [J]. Nature immunology, 2003, 4(10): 1009-15.
[20] LE BON A, DURAND V, KAMPHUIS E, et al. Direct stimulation of T cells by type I IFN enhances the CD8+ T cell response during cross-priming [J]. The Journal of Immunology, 2006, 176(8): 4682-9.
[21] SPADARO F, LAPENTA C, DONATI S, et al. IFN-α enhances cross-presentation in human dendritic cells by modulating antigen survival, endocytic routing, and processing [J]. Blood, The Journal of the American Society of Hematology, 2012, 119(6): 1407-17.
[22] PARLATO S, SANTINI S M, LAPENTA C, et al. Expression of CCR-7, MIP-3β, and Th-1 chemokines in type I IFN-induced monocyte-derived dendritic cells: importance for the rapid acquisition of potent migratory and functional activities [J]. Blood, The Journal of the American Society of Hematology, 2001, 98(10): 3022-9.
[23] ROUZAUT A, GARASA S, TEIJEIRA Á, et al. Dendritic cells adhere to and transmigrate across lymphatic endothelium in response to IFN-α [J]. European journal of immunology, 2010, 40(11): 3054-63.
[24] LE BON A, SCHIAVONI G, D'AGOSTINO G, et al. Type I interferons potently enhance humoral immunity and can promote isotype switching by stimulating dendritic cells in vivo [J]. Immunity, 2001, 14(4): 461-70.

## Claims

1. A vaccine, which comprises a fusion protein containing an interferon, a target antigen, and an immunoglobulin Fc region, as first structural unit, second structural unit, and third structural unit, respectively,
wherein the interferon is the first structural unit, which is shown in the amino acid sequence of SEQ ID NO: 1, SEQ ID NO: 11, or SEQ ID NO: 12,
wherein the immunoglobulin Fc region is the second structural unit,
wherein the target antigen is the third structural unit, which is HBV Pres1 antigen, and
wherein the fusion protein further contains one or more Th cell helper epitope(s) and linking fragments.

2. The vaccine of claim 1, wherein the fusion protein is a homodimer fusion protein comprising a first polypeptide chain and a second polypeptide chain, wherein the first polypeptide chain and the second polypeptide chain are identical, and each comprises, from N-terminal to C-terminal, the interferon, the Th cell helper epitope(s), the target antigen, and the immunoglobulin Fc region.

3. The vaccine of claim 1 or 2, wherein the immunoglobulin Fc region is selected from Fc region of IgG1, IgG2, IgG3, IgG4 and IgM, preferably Fc region of IgG1.

4. The vaccine of any one of claims 1-3, wherein the target antigen is HBV Pres1 antigen shown in the amino acid sequence of SEQ ID NO: 6, SEQ ID NO: 7, SEQ ID NO: 16, SEQ ID NO: 17, or SEQ ID NO: 18.

5. The vaccine of any one of claims 1-4, wherein the amino acid sequence of the Th cell helper epitope is shown in SEQ ID NO: 3.

6. The vaccine of any one of claims 1-5, wherein the fusion protein contains a linking fragment between each structural unit, and the linking fragment is a flexible polypeptide sequence selected from the amino acid sequences of SEQ ID NO: 4 and SEQ ID NO: 15.

7. A nucleic acid molecule encoding the fusion protein as described in any one of claims 1-6.

8. An expression vector comprising the nucleic acid molecule of claim 7.

9. A host cell, such as an eukaryotic cell, comprising the nucleic acid molecule of claim 7 or the expression vector of claim 8.

10. The fusion protein in the vaccine defined in any one of claims 1-6 for use in the prevention or treatment of HBV.

11. The vaccine of claim 10, wherein the fusion protein is in a composition or kit.

12. The vaccine of claim 11, wherein the composition or kit is used as a prophylactic or therapeutic vaccine for hepatitis B.

13. The vaccine of any one of claims 1-6 and claims 10-12, wherein the vaccine, the composition or the kit can be inoculated by intramuscular, intravenous, transdermal, subcutaneous or nasal or other immunization routes, wherein the vaccine, the composition or the kit can also comprise an adjuvant, and the adjuvant can comprise aluminum adjuvant (Alum), Toll-like receptor 4 activator ligand MPLA, Toll-like receptor 9 ligand, oligodeoxynucleotide (CpG-ODN), MF59 and Freund's adjuvant.

14. The vaccine of any one of claims 1-6 and claims 10-13, wherein the vaccine can be used in combination with other prophylactic or therapeutic therapies; for example, the vaccine can be hepatitis B therapeutic vaccine, which can be used in combination with another prophylactic or therapeutic hepatitis B therapy, for example, the hepatitis B therapeutic vaccine can be used in combination with hepatitis B virus envelope protein HBsAg vaccine, for example for the treatment of chronic hepatitis B virus infection, for example, the hepatitis B therapeutic vaccine can be combined with nucleoside or nucleotide analogues, for example for the treatment of chronic hepatitis B virus infection, for example, the vaccine can be combined with other vaccines for viruses or pathogens or tumors to form a multivalent vaccine, for example, the vaccine and an adenovirus vaccine or mRNA vaccine or inactivated vaccine or DNA vaccine for the same virus are inoculated in sequence or at the same time.

15. The fusion protein in the vaccine defined in any one of claims 1-6 for use in preventing or treating hepatitis B virus infection in a subject.
